# EUROPEAN PATENT APPLICATION

(11) **EP 4 671 366 A1**
(43) Date of publication of application: **31.12.2025**
(21) Application number: 24759673.7
(22) Date of filing: 20.02.2024
(51) Int. Cl.: C12N 5/0793, C12N 5/0797, A61K 38/18, A61K 35/30

(54) **KIT FOR INDUCING DIFFERENTIATION OF STEM CELLS INTO DOPAMINERGIC NEURAL PRECURSOR CELLS AND/OR DOPAMINE NEURONS, AND USE THEREOF**

(30) Priority: 21.02.2023 CN 202310151060
(71) Applicant: Xellsmart Biomedical (Suzhou) Co., Ltd, Suzhou, Jiangsu 210008 (CN)
(72) Inventor: LI, Xiang, Shanghai 200241 (CN); XI, Jiajie, Shanghai 200241 (CN); GUO, Wenke, Shanghai 200241 (CN); LI, Xueting, Shanghai 200241 (CN); ZHANG, Lei, Shanghai 200241 (CN)
(74) Representative: Schnappauf, Georg
(86) International application number: PCT/CN2024/077766
(87) International publication number: WO 2024/174996

(57) **Abstract**

Provided are a kit for inducing differentiation of stem cells into dopaminergic neural precursor cells and/or dopamine neurons, and a use thereof. The kit uses chemically defined culture media, chemical small molecules and cytokines, and does not involve substances such as animal-derived components which easily cause unstable factors such as batch-to-batch variation. Moreover, the differentiation period is shortened, and the kit can be used in clinical practice.

## Description

### TECHNICAL FIELD

The present disclosure pertains to the technical field of biology, and particularly relates to a kit for inducing differentiation of stem cells into dopaminergic neural precursor cells and/or dopamine neurons, and use thereof.

### BACKGROUND

Parkinson's disease (PD) is a common neurodegenerative disease in the middle-aged and elderly populations. Clinically, it is mainly manifested by typical symptoms such as resting tremor, muscular rigidity, bradykinesia, and postural instability. In addition, it is also manifested by a series of other non-motor symptoms, such as language dysfunction and psychiatric disorders like depression. The specific etiology and pathogenesis of PD are highly complex, and the main pathological features are the degeneration and loss of dopaminergic neurons in the substantia nigra pars compacta of the midbrain and insufficient dopamine secretion in the striatum. Currently, the primary clinical treatment strategies rely on conventional pharmacotherapy. However, the efficacy of drug treatment is significantly limited, only partially alleviating patients' motor symptoms without achieving fundamental therapeutic objectives. Furthermore, the efficacy of drugs gradually declines over time, frequently accompanied by severe side effects such as involuntary movements. With the development of stem cell technologies and the implementation of a large number of clinical trials involving stem cells, cell therapy has demonstrated promising efficacy in treating neurodegenerative diseases like PD, and may become the most effective treatment modality for PD.

Pluripotent stem cells (PSCs) are a class of stem cells with unlimited proliferative capacity and multilineage differentiation potential. PSCs can be induced *in vitro* to differentiate into dopaminergic neural precursor cells, serving as an ideal cell source for PD treatment. Although numerous differentiation methods currently exist, they all exhibit certain limitations, such as low differentiation efficiency, prolonged differentiation periods, and the inability to generate mature functional neurons. In addition, some differentiation methods frequently employ Matrigel as a matrix or mouse fibroblasts as a feeder layer for neural differentiation. These methods introduce animal-derived components, rendering them unsuitable for clinical applications.

### SUMMARY

In a first aspect, the objective of the present disclosure is to provide a kit for inducing differentiation of stem cells into dopaminergic neural precursor cells or dopamine neurons.

In a second aspect, the objective of the present disclosure is to provide use of the kit according to the first aspect.

In a third aspect, the objective of the present disclosure is to provide a method for inducing differentiation of stem cells into dopaminergic neural precursor cells or dopamine neurons.

In a fourth aspect, the objective of the present disclosure is to provide a dopaminergic neural precursor cell.

In a fifth aspect, the objective of the present disclosure is to provide a dopamine neuron.

In a sixth aspect, the objective of the present disclosure is to provide a pharmaceutical composition.

In a seventh aspect, the objective of the present disclosure is to provide use of the dopaminergic neural precursor cell according to the fourth aspect of the present disclosure, the dopamine neuron according to the fifth aspect of the present disclosure, and/or the pharmaceutical composition according to the sixth aspect of the present disclosure.

To achieve the aforementioned objectives, the present disclosure adopts the following technical solutions:
In a first aspect of the present disclosure, provided is a kit for inducing differentiation of stem cells into dopaminergic neural precursor cells. The kit includes a first culture medium, a second culture medium, a third culture medium, a fourth culture medium, and a fifth culture medium.

The first culture medium is a basal culture medium including a GSK3β inhibitor, an SHH agonist, and at least one or at least two of the following: a TGFβ/ALK inhibitor and a BMP4 inhibitor.

The second culture medium is a basal culture medium including a GSK3β inhibitor, an SHH agonist, and at least one, at least two, or at least three of the following: a ROCK inhibitor, a TGFβ/ALK inhibitor, and a BMP4 inhibitor.

The third culture medium is a basal culture medium including at least one, at least two, or at least three of the following: a BMP4 inhibitor, an SHH agonist, and a growth factor.

The fourth culture medium is a basal culture medium including at least one or at least two of the following: an SHH agonist and a growth factor.

The fifth culture medium is a basal culture medium including at least one, at least two, or at least three of the following: a ROCK inhibitor, an SHH agonist, and a growth factor.

Preferably, the first culture medium is a basal culture medium including a TGFβ/ALK inhibitor, a BMP4 inhibitor, a GSK3β inhibitor, and an SHH agonist.

Preferably, the second culture medium is a basal culture medium including a ROCK inhibitor, a TGFβ/ALK inhibitor, a BMP4 inhibitor, a GSK3β inhibitor, and an SHH agonist.

Preferably, the third culture medium is a basal culture medium including a BMP4 inhibitor, an SHH agonist, and a growth factor.

Preferably, the fourth culture medium is a basal culture medium including an SHH agonist and a growth factor.

Preferably, the fifth culture medium is a basal culture medium including a ROCK inhibitor, an SHH agonist, and a growth factor.

Preferably, the TGFβ/ALK inhibitors of the first culture medium and the second culture medium are each independently selected from at least one of: SB431542, SB-505, A-83-01, GW6604, IN-1130, Ki26894, LY2157299, LY364947 (HTS-466284), LY550410, LY573636, LY580276, NPC-30345, SB-505124, SD-093, Sm16, SM305, SX-007, Antp-Sm2A, and LY2109761.

Preferably, the TGFβ/ALK inhibitor of the first culture medium includes SB431542.

Preferably, the concentration of the TGFβ/ALK inhibitor in the first culture medium is 0.2 µM to 100 µM, further preferably 1 µM to 20 µM, and still further preferably 10 µM.

Preferably, the TGFβ/ALK inhibitor of the second culture medium includes SB431542.

Preferably, the concentration of the TGFβ/ALK inhibitor in the second culture medium is 0.2 µM to 100 µM, further preferably 1 µM to 20 µM, and still further preferably 10 µM.

Preferably, the BMP4 inhibitors of the first culture medium, the second culture medium, and the third culture medium are each independently selected from: at least one of dorsomorphin, noggin, LDN-193189, follistatin, chordin, gremlin, and DMH1.

Preferably, the BMP4 inhibitor of the first culture medium includes DMH1.

Preferably, the concentration of the BMP4 inhibitor in the first culture medium is 0.2 µM to 10 µM, further preferably 1 µM to 3 µM, and still further preferably 2 µM.

Preferably, the BMP4 inhibitor of the second culture medium includes DMH1.

Preferably, the concentration of the BMP4 inhibitor in the second culture medium is 0.2 µM to 10 µM, further preferably 1 µM to 3 µM, and still further preferably 2 µM.

Preferably, the BMP4 inhibitor of the third culture medium includes DMH1.

Preferably, the concentration of the BMP4 inhibitor in the third culture medium is 0.2 µM to 10 µM, further preferably 0.8 µM to 1.2 µM, and still further preferably 1 µM.

Preferably, the GSK3β inhibitors of the first culture medium and the second culture medium are each independently selected from: at least one of GSK3β inhibitor IX (6-bromoindirubin-3'-oxime), SB216763, GSK3β inhibitor VII (4-dibromoacetophenone), L803-mts, 6-bromo-indirubin-3'-oxime (BIO), TWS119, AZD2858, AR-A014418, TDZD-8, LY2090314, 2-D08, IM-12, 1-azakenpaullone, indirubin, and CHIR99021.

Preferably, the GSK3β inhibitor of the first culture medium includes CHIR99021.

Preferably, the concentration of the GSK3β inhibitor in the first culture medium is 0.2 µM to 3 µM, further preferably 0.2 µM to 1 µM, and still further preferably 0.6 µM.

Further preferably, the GSK3β inhibitor in the second culture medium includes CHIR99021.

Preferably, the concentration of the GSK3β inhibitor in the second culture medium is 0.2 µM to 3 µM, further preferably 0.2 µM to 1 µM, and still further preferably 0.6 µM.

Preferably, the SHH agonists of the first culture medium, the second culture medium, the third culture medium, the fourth culture medium, and the fifth culture medium are each independently selected from: at least one of SHH, SHH C25II, SAG, SAG 21K, Hh-Ag1.5, 20a-hydroxycholesterol, and purmorphamine.

Preferably, the SHH agonist of the first culture medium includes SAG.

Preferably, the concentration of the SHH agonist in the first culture medium is 0.5 µM to 2.5 µM, further preferably 1 µM to 1.75 µM, and still further preferably 1 µM.

Preferably, the SHH agonist of the second culture medium includes SAG.

Preferably, the concentration of the SHH agonist in the second culture medium is 0.5 µM to 2.5 µM, further preferably 1 µM to 1.75 µM, and still further preferably 1 µM.

Preferably, the SHH agonist of the third culture medium includes SAG.

Preferably, the concentration of the SHH agonist in the third culture medium is 0.1 µM to 1 µM, further preferably 0.1 µM to 0.5 µM, and still further preferably 0.2 µM.

Preferably, the SHH agonist of the fourth culture medium includes SAG.

Preferably, the concentration of the SHH agonist in the fourth culture medium is 0.1 µM to 1 µM, further preferably 0.1 µM to 0.5 µM, and still further preferably 0.2 µM.

Preferably, the SHH agonist of the fifth culture medium includes SAG.

Preferably, the concentration of the SHH agonist in the fifth culture medium is 0.1 µM to 1 µM, further preferably 0.1 µM to 0.5 µM, and still further preferably 0.2 µM.

Preferably, the ROCK inhibitors of the second culture medium and the fifth culture medium are each independently selected from: at least one of Y-27632, HA100, HA1152, blebbistatin, HA-1077, KD-025, Y-33075, and narciclasine.

Further preferably, the ROCK inhibitor of the second culture medium includes Y-27632.

Preferably, the concentration of the ROCK inhibitor in the second culture medium is 5 µM to 15 µM, further preferably 10 µM.

Further preferably, the ROCK inhibitor of the fifth culture medium includes Y-27632.

Preferably, the concentration of the ROCK inhibitor in the fifth culture medium is 5 µM to 15 µM, further preferably 10 µM.

Preferably, the growth factors of the third culture medium, the fourth culture medium, and the fifth culture medium are each independently selected from: at least one of epidermal growth factor (EGF), platelet-derived growth factor (PDGF), fibroblast growth factor (FGF),

hepatocyte growth factor (HGF), insulin-like growth factor-I (IGF-1), IGF-II, leukemia inhibitory factor (LIF), nerve growth factor (NGF), oncostatin M (OSM), platelet-derived endothelial cell growth factor (PDECGF), transforming growth factor-α (TGF-α), and vascular endothelial cell growth factor (VEGF).

Further preferably, the growth factor of the third culture medium includes fibroblast growth factor.

Preferably, the concentration of the growth factor in the third culture medium is 10 ng/mL to 1 mg/mL, further preferably 80 ng/mL to 120 ng/mL, and still further preferably 100 ng/mL.

Further preferably, the growth factor of the fourth culture medium includes fibroblast growth factor.

Preferably, the concentration of the growth factor in the fourth culture medium is 10 ng/mL to 1 mg/mL, further preferably 80 ng/mL to 120 ng/mL, and still further preferably 100 ng/mL.

Further preferably, the growth factor of the fifth culture medium includes fibroblast growth factor.

Preferably, the concentration of the growth factor in the fifth culture medium is 10 ng/mL to 1 mg/mL, further preferably 80 ng/mL to 120 ng/mL, and still further preferably 100 ng/mL.

Still further preferably, the fibroblast growth factor includes FGF8b.

Preferably, the basal culture media of the first culture medium, the second culture medium, the third culture medium, the fourth culture medium, and the fifth culture medium are each independently selected from at least one of Iscove's Modified Dulbecco's Medium (IMDM), Eagle's Basal Medium (BME), GMEM, MEM, DMEM, Ham's F-12 Medium, RPMI1640 Medium, and Neurobasal Medium.

Preferably, the basal media of the first culture medium, the second culture medium, the third culture medium, the fourth culture medium, and the fifth culture medium are basal media including a non-essential amino acid and an N2 supplement.

Preferably, the concentration of the non-essential amino acid in the basal culture media is 0.2% to 5% (v/v), further preferably 0.5% to 2% (v/v), and still further preferably 1% (v/v).

Preferably, the concentration of the N2 supplement in the basal culture media is 0.2% to 5% (v/v), further preferably 0.5% to 2% (v/v), and still further preferably 1% (v/v).

Preferably, the basal culture medium of the first culture medium includes DMEM/F12.

Preferably, the basal culture medium of the second culture medium includes DMEM/F12.

Preferably, the basal culture medium of the third culture medium includes DMEM/F12.

Preferably, the basal culture medium of the fourth culture medium includes DMEM/F12.

Preferably, the basal culture medium of the fifth culture medium includes DMEM/F12.

In the present disclosure, the first culture medium and the second culture medium are used for inducing differentiation of stem cells into midbrain floor plate cells.

In the present disclosure, the third culture medium is used for inducing differentiation of the midbrain floor plate cells into dopaminergic neural progenitor cells.

In the present disclosure, the fourth culture medium and the fifth culture medium are used for inducing differentiation of the dopaminergic neural progenitor cells into dopaminergic neural precursor cells.

Preferably, the stem cells are stem cells with multilineage differentiation potential.

Preferably, the stem cells with multilineage differentiation potential include at least one of embryonic stem cells, parthenogenetic stem cells, induced pluripotent stem cells, mesenchymal stem cells, adipose-derived stem cells, and cord blood stem cells; further preferably, the stem cells with multilineage differentiation potential include induced pluripotent stem cells.

Preferably, the stem cells are derived from a mammal, further preferably from a primate, and still further preferably from a human.

Preferably, the stem cells are human embryonic stem cells (hESCs) (e.g., H1 and H9) and/or human induced pluripotent stem cells (hiPSCs) (e.g., WC50 and IMR90).

Preferably, the human embryonic stem cells are commercially available human embryonic stem cell lines.

Preferably, the human embryonic stem cells are stem cells isolated or obtained from a human embryo within 14 days after fertilization without *in vivo* development. Provided is a kit for inducing differentiation of stem cells into dopamine neurons, including: the aforementioned kit for inducing differentiation of stem cells into dopaminergic neural precursor cells and a sixth culture medium. The sixth culture medium is a basal culture medium including a brain-derived growth factor (BDNF), a glial cell line-derived neurotrophic factor (GDNF), ascorbic acid (AA), cyclic adenosine monophosphate (cAMP), and compound E.

Preferably, the concentration of the brain-derived growth factor (BDNF) in the sixth culture medium is 1 ng/mL to 100 ng/mL, further preferably 15 ng/mL to 25 ng/mL, and still further preferably 20 ng/mL.

Preferably, the concentration of the glial cell line-derived neurotrophic factor (GDNF) in the sixth culture medium is 1 ng/mL to 100 ng/mL, further preferably 15 ng/mL to 25 ng/mL, and still further preferably 20 ng/mL.

Preferably, the concentration of the ascorbic acid (AA) in the sixth culture medium is 10 µM to 1 mM, further preferably 150 µM to 250 µM, and still further preferably 200 µM.

Preferably, the concentration of the cyclic adenosine monophosphate (cAMP) in the sixth culture medium is 0.1 µM to 10 µM, further preferably 0.5 µM to 1.5 µM, and still further preferably 1 µM.

Preferably, the concentration of the compound E in the sixth culture medium is 0.05 µM to 1 µM, further preferably 0.05 µM to 0.15 µM, and still further preferably 0.1 µM.

Preferably, the basal culture medium of the sixth culture medium includes at least one of Iscove's Modified Dulbecco's Medium (IMDM), Eagle's Basal Medium (BME), GMEM, MEM, DMEM, Ham's F-12 Medium, RPMI1640 Medium, and Neurobasal Medium. Further preferably, the basal culture medium of the sixth culture medium includes Neurobasal Medium.

Preferably, the basal culture medium of the sixth culture medium is a basal culture medium including an N2 supplement, a non-essential amino acid, and a B27 supplement.

Preferably, the concentration of the B27 supplement in the basal culture medium is 0.2% to 5% (v/v), further preferably 0.5% to 2% (v/v), and still further preferably 1% (v/v).

Preferably, the concentration of the non-essential amino acid in the basal culture medium is 0.2% to 5% (v/v), further preferably 0.5% to 2% (v/v), and still further preferably 1% (v/v).

Preferably, the concentration of the N2 supplement in the basal culture medium is 0.2% to 5% (v/v), further preferably 0.5% to 2 % (v/v), and still further preferably 0.5% (v/v).

In the present disclosure, the sixth culture medium is used for inducing differentiation of dopaminergic neural precursor cells into dopamine neurons.

Preferably, the kit for inducing differentiation of stem cells into dopaminergic neural precursor cells further includes a seventh culture medium, and the seventh culture medium is a basal culture medium including a ROCK inhibitor.

Preferably, the concentration of the ROCK inhibitor in the seventh culture medium is 5 µM to 15 µM, further preferably 10 µM.

Preferably, the basal culture medium of the seventh culture medium includes at least one of Iscove's Modified Dulbecco's Medium (IMDM), Eagle's Basal Medium (BME), MEM, DMEM, Ham's F-12 Medium, RPMI1640 Medium, Fischer's Medium, E8 Medium, mTESR Medium, StemFit Basic 03, StemFit Basic 04, NutriStem hPSC XF Medium, StemMACS iPS Brew Medium, Stem-Partner ACF Medium, TeSR-AOF Medium, TeSR2 Medium, and StemFit Complete Medium, and further includes StemFit Complete Medium.

In the present disclosure, the seventh culture medium is used for promoting the survival of stem cells.

In a second aspect of the present disclosure, provided is use of the kit according to the first aspect of the present disclosure in any one of (1) to (4):
(1) preparing dopaminergic neural precursor cells; (2) preparing dopamine neurons; (3) preparing a product for inducing differentiation of stem cells into dopaminergic neural precursor cells; and (4) preparing a product for inducing differentiation of stem cells into dopamine neurons.

Preferably, the stem cells are stem cells with multilineage differentiation potential.

Preferably, the stem cells with multilineage differentiation potential include at least one of embryonic stem cells, parthenogenetic stem cells, induced pluripotent stem cells, mesenchymal stem cells, adipose-derived stem cells, and cord blood stem cells; further preferably, the stem cells with multilineage differentiation potential include induced pluripotent stem cells.

Preferably, the stem cells are derived from a mammal, further preferably from a primate, and still further preferably from a human.

Preferably, the stem cells are human embryonic stem cells (hESCs) (e.g., H1 and H9) and/or human induced pluripotent stem cells (hiPSCs) (e.g., WC50 and IMR90).

Preferably, the human embryonic stem cells are commercially available human embryonic stem cell lines.

Preferably, the human embryonic stem cells are stem cells isolated or obtained from a human embryo within 14 days after fertilization without *in vivo* development. In a third aspect of the present disclosure, provided is a method for inducing differentiation of stem cells into dopaminergic neural precursor cells. The method includes a step of using the kit for inducing differentiation of stem cells into dopaminergic neural precursor cells and/or the kit for inducing differentiation of stem cells into dopamine neurons according to the first aspect of the present disclosure.

Preferably, the method includes the following steps:
(1) subjecting the stem cells to a first culture using the first culture medium in the kit according to the first aspect of the present disclosure, then resuspending the stem cells in the second culture medium in the kit according to the first aspect of the present disclosure, and performing a second culture to obtain midbrain floor plate cells;
(2) subjecting the midbrain floor plate cells to a third culture using the third culture medium in the kit according to the first aspect of the present disclosure to obtain dopaminergic neural progenitor cells; and
(3) subjecting the dopaminergic neural progenitor cells to a fourth culture using the fourth culture medium in the kit according to the first aspect of the present disclosure, and then to a fifth culture using the fifth culture medium in the kit according to the first aspect of the present disclosure to obtain dopaminergic neural precursor cells.

Preferably, the first culture in step (1) is performed for 6 to 10 days, further preferably 7 to 9 days, and still further preferably 8 days.

Preferably, the first culture in step (1) is adherence culture, further preferably adherence culture on a culture device coated with Vitronectin.

Preferably, the second culture in step (1) is performed for 1 to 3 days, further preferably 1.5 to 2.5 days, and still further preferably 2 days.

Preferably, step (1) further includes a dissociation step before the second culture.

Preferably, the second culture in step (1) is suspension culture.

Preferably, the third culture in step (2) is performed for 3 to 7 days, further preferably 4 to 6 days, and still further preferably 5 days.

Preferably, the third culture in step (2) is suspension culture.

Preferably, the fourth culture in step (3) is performed for 5 to 9 days, further preferably 6 to 8 days, and still further preferably 7 days.

Preferably, the fourth culture in step (3) is adherence culture, further preferably adherence culture on a culture device coated with Vitronectin.

Preferably, the fifth culture in step (3) is performed for 0.5 to 1.5 days, further preferably 1 day.

Preferably, step (3) further includes a dissociation step before the fifth culture.

Preferably, the fifth culture in step (3) is suspension culture.

Preferably, before the first culture of the stem cells in step (1), the method further includes the following step: performing an N-th culture using the seventh culture medium in the kit according to the first aspect of the present disclosure.

Preferably, the N-th culture is performed for 0.5 to 1.5 days, further preferably 1 day.

Preferably, the culture described above is performed at 32 to 38 °C with 4 to 6% CO₂.

Provided is a method for inducing differentiation of stem cells into dopamine neurons. The method includes a step of using the kit for inducing differentiation of stem cells into dopamine neurons according to the first aspect of the present disclosure.

Preferably, the method includes the following steps:
(1) subjecting the stem cells to a first culture using the first culture medium in the kit according to the first aspect of the present disclosure, then resuspending the stem cells in the second culture medium in the kit according to the first aspect of the present disclosure, and performing a second culture to obtain midbrain floor plate cells;
(2) subjecting the midbrain floor plate cells to a third culture using the third culture medium in the kit according to the first aspect of the present disclosure to obtain dopaminergic neural progenitor cells;
(3) subjecting the dopaminergic neural progenitor cells to a fourth culture using the fourth culture medium in the kit according to the first aspect of the present disclosure, and then to a fifth culture using the fifth culture medium in the kit according to the first aspect of the present disclosure to obtain dopaminergic neural precursor cells; and
(4) subjecting the dopaminergic neural precursor cells to a sixth culture using the sixth culture medium in the kit according to the first aspect of the present disclosure to obtain dopamine neurons.

Preferably, the first culture in step (1) is performed for 6 to 10 days, further preferably 7 to 9 days, and still further preferably 8 days.

Preferably, the first culture in step (1) is adherence culture, further preferably adherence culture on a culture device coated with Vitronectin.

Preferably, the second culture in step (1) is performed for 1 to 3 days, further preferably 1.5 to 2.5 days, and still further preferably 2 days.

Preferably, step (1) further includes a dissociation step before the second culture.

Preferably, the second culture in step (1) is suspension culture.

Preferably, the third culture in step (2) is performed for 3 to 7 days, further preferably 4 to 6 days, and still further preferably 5 days.

Preferably, the third culture in step (2) is suspension culture.

Preferably, the fourth culture in step (3) is performed for 5 to 9 days, further preferably 6 to 8 days, and still further preferably 7 days.

Preferably, the fourth culture in step (3) is adherence culture, further preferably adherence culture on a culture device coated with Vitronectin.

Preferably, the fifth culture in step (3) is performed for 0.5 to 1.5 days, further preferably 1 day.

Preferably, step (3) further includes a dissociation step before the fifth culture.

Preferably, the fifth culture in step (3) is suspension culture.

Preferably, the sixth culture in step (4) is performed for 18 to 24 days, further preferably 20 to 22 days, and still further preferably 21 days.

Preferably, step (4) further includes a dissociation step before the sixth culture.

Preferably, the sixth culture in step (4) is adherence culture, further preferably adherence culture on a culture device coated with Matrigel.

Preferably, before the first culture of the stem cells in step (1), the method further includes the following step: performing an N-th culture using the seventh culture medium in the kit according to the first aspect of the present disclosure.

Preferably, the N-th culture is performed for 0.5 to 1.5 days, further preferably 1 day.

Preferably, the culture described above is performed at 32 to 38 °C with 4 to 6% CO₂.

Preferably, the stem cells are stem cells with multilineage differentiation potential.

Preferably, the stem cells with multilineage differentiation potential include at least one of embryonic stem cells, parthenogenetic stem cells, induced pluripotent stem cells, mesenchymal stem cells, adipose-derived stem cells, and cord blood stem cells; further preferably, the stem cells with multilineage differentiation potential include induced pluripotent stem cells.

Preferably, the stem cells are derived from a mammal, further preferably from a primate, and still further preferably from a human.

Preferably, the stem cells are human embryonic stem cells (hESCs) (e.g., H1 and H9) and/or human induced pluripotent stem cells (hiPSCs) (e.g., WC50 and IMR90).

Preferably, the human embryonic stem cells are commercially available human embryonic stem cell lines.

Preferably, the human embryonic stem cells are stem cells isolated or obtained from a human embryo within 14 days after fertilization without *in vivo* development. In a fourth aspect of the present disclosure, provided is a dopaminergic neural precursor cell. The cell is prepared using the kit for inducing differentiation of stem cells into dopaminergic neural precursor cells and/or the kit for inducing differentiation of stem cells into dopamine neurons according to the first aspect of the present disclosure, and/or the method for inducing differentiation of stem cells into dopaminergic neural precursor cells according to the third aspect of the present disclosure.

Preferably, the content of OTX2+EN1+ cells in the dopaminergic neural precursor cell is 80% or more, 85% or more, 90% or more, 91% or more, 92% or more, 93% or more, 94% or more, 95% or more, or 96% or more, further preferably 90.1%.

Preferably, the content of FOXA2+LMX1A+ cells in the dopaminergic neural precursor cell is 80% or more, 85% or more, 90% or more, 91% or more, 92% or more, 93% or more, 94% or more, 95% or more, or 96% or more, further preferably 87.5%.

Preferably, the content of CD166+ cells in the dopaminergic neural precursor cell is 80% or more, 85% or more, 90% or more, 91% or more, 92% or more, 93% or more, 94% or more, 95% or more, or 96% or more, further preferably 97.07%.

Preferably, the content of Corin+ cells in the dopaminergic neural precursor cell is 80% or more, 85% or more, 90% or more, 91% or more, 92% or more, 93% or more, 94% or more, 95% or more, or 96% or more, further preferably 87.74%.

Preferably, the content of CD166+Corin+ cells in the dopaminergic neural precursor cell is 80% or more, 85% or more, 90% or more, 91% or more, 92% or more, 93% or more, 94% or more, 95% or more, or 96% or more, further preferably 82.42%.

Preferably, the dopaminergic neural precursor cell includes a population of dopaminergic neural precursor cells.

In a fifth aspect of the present disclosure, provided is a dopamine neuron. The dopamine neuron is prepared using the kit for inducing differentiation of stem cells into dopamine neurons according to the first aspect of the present disclosure and/or the method for inducing differentiation of stem cells into dopamine neurons according to the third aspect of the present disclosure.

Preferably, the dopamine neuron includes a dopamine neuron population.

In a sixth aspect of the present disclosure, provided is a pharmaceutical composition. The pharmaceutical composition includes the dopaminergic neural precursor cell according to the fourth aspect of the present disclosure and/or the dopamine neuron according to the fifth aspect of the present disclosure.

Preferably, the pharmaceutical composition further includes a pharmaceutically acceptable carrier or auxiliary material.

Preferably, the pharmaceutical composition is a liquid formulation.

Preferably, the pharmaceutically acceptable carrier or auxiliary material includes, but is not limited to: at least one of normal saline, water, cell buffer, cerebrospinal fluid, compound electrolyte injection, Ringer's solution, and trehalose solution.

Preferably, the water is ddH₂O.

Preferably, the cell buffer includes: PBS, HBSS, EBSS, HEPES, and other common cell buffers; further preferably, the cell buffer includes: at least one of PBS and HBSS.

Preferably, the cerebrospinal fluid is an artificial cerebrospinal fluid.

Preferably, the pharmaceutical composition further includes an instruction.

Preferably, the instruction describes the administration dose and/or the number of administrations of the dopaminergic neural precursor cell and/or the dopamine neuron.

Preferably, the administration dose of the dopaminergic neural precursor cell and/or the dopamine neuron is 1 × 10⁵ to 4 × 10⁸ cells per administration, further preferably 2 × 10⁵ to 2 × 10⁸ cells per administration.

Preferably, the dopaminergic neural precursor cell and/or the dopamine neuron are administered 1 to 3 times.

Preferably, the pharmaceutical composition further includes other drugs for preventing and/or treating a nervous system disease.

Preferably, the nervous system disease includes at least one of neurodegenerative disease and nerve injury disease; further preferably, the nervous system disease includes at least one of Parkinson's disease, amyotrophic lateral sclerosis, Alzheimer's disease, Huntington's chorea, spinal cord injury, cerebral stroke, intracranial tumor, epilepsy, craniocerebral trauma, hereditary nervous system disease, cerebrovascular disease, paralysis, myasthenia gravis, multiple sclerosis, peripheral nervous system disease, neuritis, cerebral palsy, and depression; still further preferably, the nervous system disease includes at least one of Parkinson's disease, amyotrophic lateral sclerosis, cerebral stroke, craniocerebral trauma, Alzheimer's disease, and spinal cord injury; yet still further preferably, the nervous system disease includes at least one of Parkinson's disease, amyotrophic lateral sclerosis, cerebral stroke, and craniocerebral trauma.

In a seventh aspect of the present disclosure, provided is use of the dopaminergic neural precursor cell according to the fourth aspect of the present disclosure, the dopamine neuron

according to the fifth aspect of the present disclosure, and the pharmaceutical composition according to the sixth aspect of the present disclosure.

Provided is use of the dopaminergic neural precursor cell according to the fourth aspect of the present disclosure and/or the dopamine neuron according to the fifth aspect of the present disclosure in constructing a cell bank.

Provided is use of the dopaminergic neural precursor cell according to the fourth aspect of the present disclosure, the dopamine neuron according to the fifth aspect of the present disclosure, and/or the pharmaceutical composition according to the sixth aspect of the present disclosure in preparing a medicament for preventing and/or treating a nervous system disease.

Preferably, the medicament further includes an instruction.

Preferably, the instruction describes the administration dose and/or the number of administrations of the dopaminergic neural precursor cell, the dopamine neuron, and/or the pharmaceutical composition.

Preferably, the administration dose of the dopaminergic neural precursor cell, the dopamine neuron, and/or the pharmaceutical composition, based on the dopaminergic neural precursor cell and/or the dopamine neuron, is 1 × 10⁵ to 4 × 10⁸ cells per administration, further preferably 2 × 10⁵ to 2 × 10⁸ cells per administration.

Preferably, the dopaminergic neural precursor cell, the dopamine neuron, and/or the pharmaceutical composition are administered 1 to 3 times.

Preferably, provided is a method for treating a nervous system disease. The method includes the following step: administering to a subject the dopaminergic neural precursor cell according to the fourth aspect of the present disclosure, the dopamine neuron according to the fifth aspect of the present disclosure, and/or the pharmaceutical composition according to the sixth aspect of the present disclosure.

Preferably, the administration dose of the dopaminergic neural precursor cell, the dopamine neuron, and/or the pharmaceutical composition, based on the dopaminergic neural precursor cell and/or the dopamine neuron, is 1 × 10⁵ to 4 × 10⁸ cells per administration, further preferably 2 × 10⁵ to 2 × 10⁸ cells per administration.

Preferably, the dopaminergic neural precursor cell, the dopamine neuron, and/or the pharmaceutical composition are administered 1 to 3 times.

Preferably, the subject is a mammal, more preferably a primate, and even more preferably a human.

Preferably, the nervous system disease includes at least one of neurodegenerative disease and nerve injury disease; further preferably, the nervous system disease includes at least one of Parkinson's disease, amyotrophic lateral sclerosis, Alzheimer's disease, Huntington's chorea, spinal cord injury, cerebral stroke, intracranial tumor, epilepsy, craniocerebral trauma, hereditary nervous system disease, cerebrovascular disease, paralysis, myasthenia gravis, multiple sclerosis, peripheral nervous system disease, neuritis, cerebral palsy, and depression; still further preferably, the nervous system disease includes at least one of Parkinson's disease, amyotrophic lateral sclerosis, cerebral stroke, craniocerebral trauma, Alzheimer's disease, and spinal cord injury; yet still further preferably, the nervous system disease includes at least one of Parkinson's disease, amyotrophic lateral sclerosis, cerebral stroke, and craniocerebral trauma.

The present disclosure has the following beneficial effects:
The present disclosure provides a kit that employs culture media, small chemical molecules, and cytokines with well-defined chemical compositions, without involving animal-derived components or other substances that are prone to cause instability such as batch-to-batch variability. In addition, the kit is less expensive compared to known commercial differentiation media, shortens the differentiation period, and is suitable for clinical application. It addresses the shortcomings of reagents used in existing differentiation methods. The dopaminergic neural precursor cells and the dopamine neurons prepared using the kit exhibit high purity and are suitable for cell transplantation and the treatment of related diseases.

### BRIEF DESCRIPTION OF THE DRAWINGS

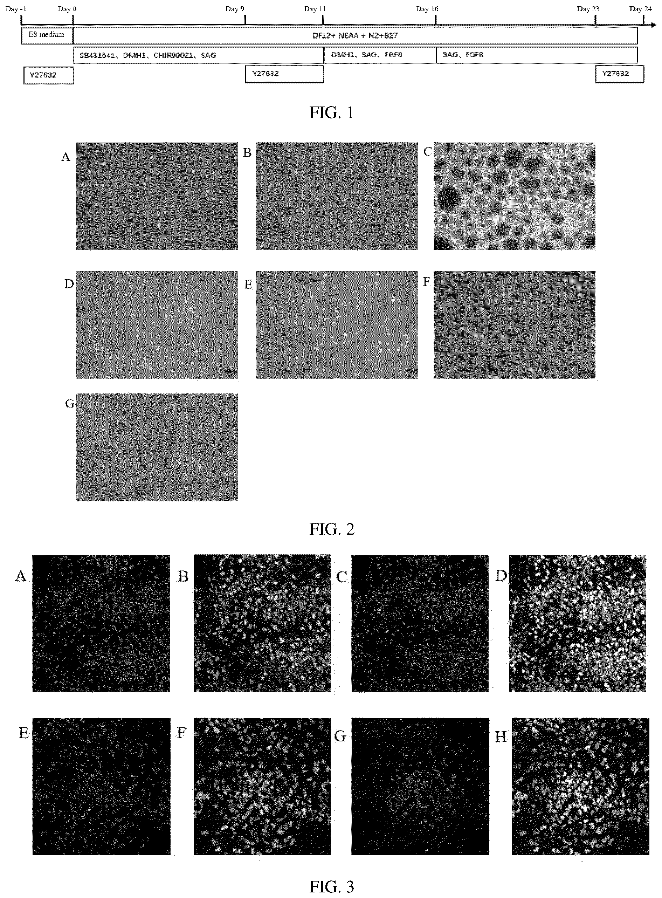

FIG. 1 is a schematic diagram of the differentiation of human pluripotent stem cells into dopaminergic neural precursor cells and dopamine neurons.
FIG. 2 is images of cell morphologies at different stages in the method of Example 7, where A illustrates the cell morphology on D0 (day 0 of differentiation); B illustrates the cell morphology on D7; C illustrates the cell morphology on D15; D illustrates the cell morphology on D20; E illustrates the cell morphology on D24; F illustrates the cell morphology on D26; G illustrates the cell morphology on D40; the scale bar is 200 µm.
FIG. 3 is immunofluorescence staining images of dopaminergic neural precursor cells obtained after induction in Example 7, where A is a DAPI immunofluorescence staining image; B is an OTX2 immunofluorescence staining image; C is an EN1 immunofluorescence staining image; D is a mixture of immunofluorescence staining images in the same field of view of A, B, and C; E is a DAPI immunofluorescence staining image; F is a FOXA2 immunofluorescence staining image; G is a LMX1A immunofluorescence staining image; H is a mixture of immunofluorescence staining images in the same field of view of E, F, and G; the scale bar is 100 µm.

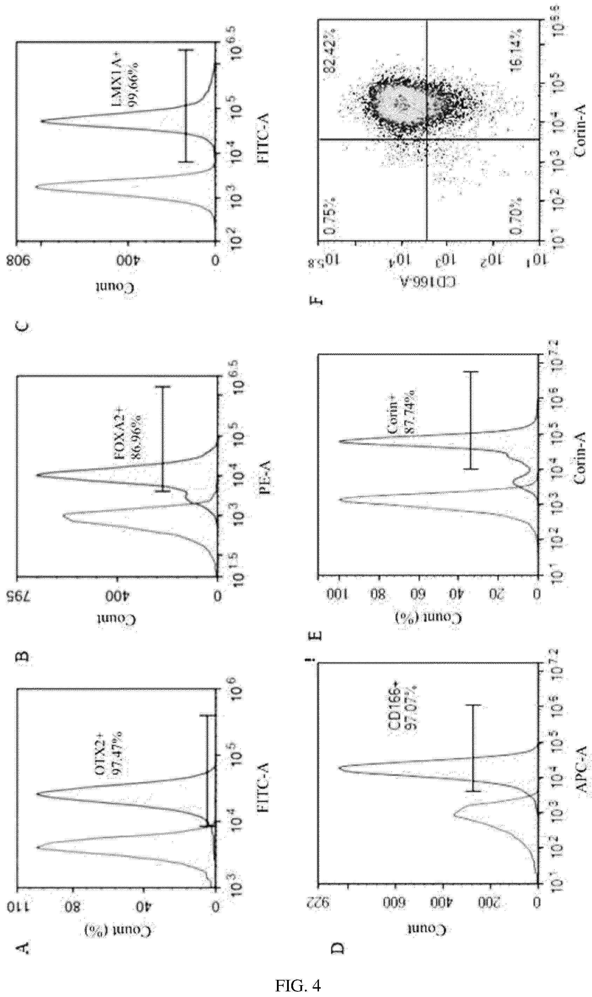

FIG. 4 is flow cytometric graphs of dopaminergic neural precursor cells obtained after induction in Example 7, where A is a cytometric graph of OTX2+ cells; B is a cytometric graph of FOXA2+ cells; C is a cytometric graph of LMX1A+ cells; D is a cytometric graph of CD166+ cells; E is a cytometric graph of Corin+ cells; F is a cytometric graph of CD166+Corin+ cells.

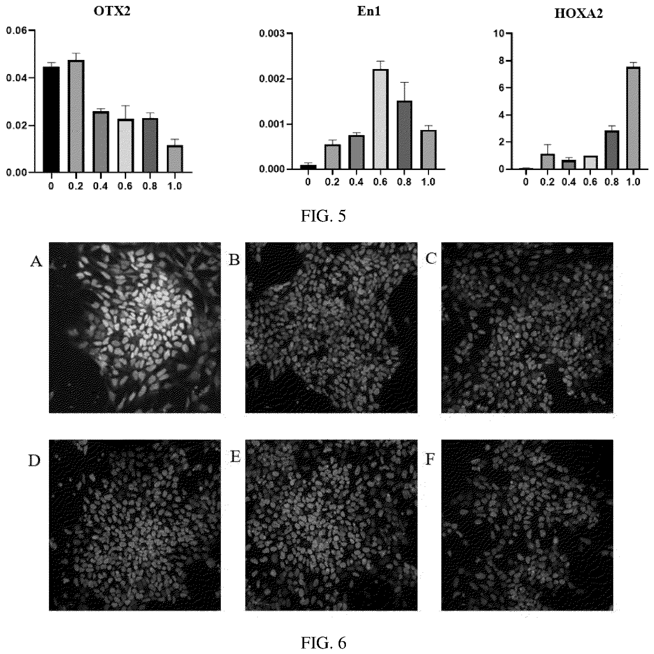

FIG. 5 is a graph illustrating relative expression levels of forebrain, midbrain, and hindbrain markers (OTX2, En1, and HOXA2) by fluorogenic quantitative PCR in midbrain floor plate cells obtained through differentiation induced by different concentrations of CHIR99021 (0 µM, 0.2 µM, 0.4 µM, 0.6 µM, 0.8 µM, and 1.0 µM, corresponding to Comparative Example 5 and Examples 8-1, 8-2, 7, 8-3, and 8-4, respectively).
FIG. 6 is immunofluorescence staining images of midbrain floor plate cells obtained through differentiation induced by different concentrations of SAG (0 µM, 0.5 µM, 1 µM, 1.25 µM, 1.5 µM and 1.75 µM, corresponding to Comparative Example 6 and Examples 9-1, 7, 9-2, 9-3 and 9-4, respectively), where A is an immunofluorescence staining image of midbrain floor plate cells obtained through differentiation induced by 0 µM SAG; B is an immunofluorescence staining image of midbrain floor plate cells obtained through differentiation induced by 0.5 µM SAG; C is an immunofluorescence staining image of midbrain floor plate cells obtained through differentiation induced by 1 µM SAG; D is an immunofluorescence staining image of midbrain floor plate cells obtained through differentiation induced by 1.25 µM SAG; E is an immunofluorescence staining image of midbrain floor plate cells obtained through differentiation induced by 1.5 µM SAG; F is an immunofluorescence staining image of midbrain floor plate cells obtained through differentiation induced by 1.75 µM SAG; red fluorescence represents mDAP cell marker FOXA2, blue fluorescence represents DAPI, green fluorescence represents PAX6; the scale bar is 100 µm.

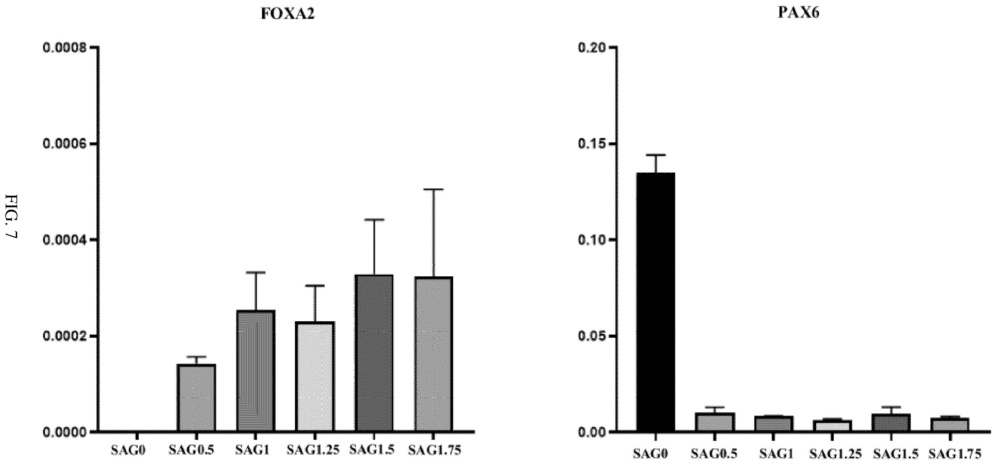

FIG. 7 is a graph illustrating relative expression levels of markers (FOXA2 and PAX6) by fluorogenic quantitative PCR in midbrain floor plate cells obtained through differentiation induced by different concentrations of SAG (0 µM, 0.5 µM, 1 µM, 1.25 µM, 1.5 µM, and 1.75 µM, corresponding to Comparative Example 6 and Examples 9-1, 7, 9-2, 9-3, and 9-4, respectively).

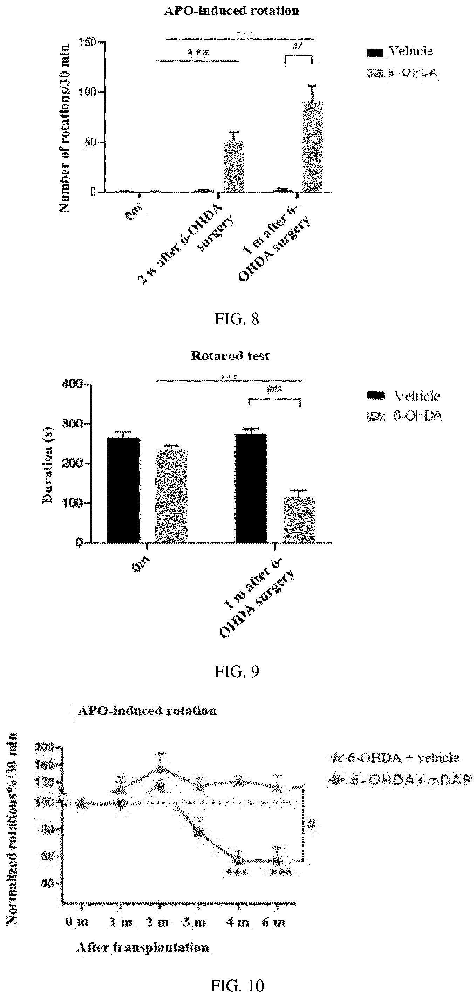

FIG. 8 is a graph illustrating APO rotation results of mice in 6-OHDA (model group) and vehicle (1% VC) group (control group) on week 4 after surgery (1 m after surgery), where *** and *** represent p < 0.001 (the difference between 6-OHDA (model group) at 0 m and 2 W after 6-OHDA surgery; the difference between 6-OHDA (model group) at 0 m and 1 m after 6-OHDA surgery); ## represents p < 0.01.
FIG. 9 is a graph illustrating rotarod test results of mice in 6-OHDA (model group) and vehicle (1% VC) group (control group) on week 4 after surgery (1 m after surgery), where *** (the difference between 6-OHDA (model group) at 0 m and 1 m after 6-OHDA surgery), and ### represent p < 0.001.
FIG. 10 is a line graph illustrating APO rotation results before cell transplantation (1 m after modeling) and 1 m, 2 m, 3 m, 4 m, and 6 m after cell transplantation, where *** (the difference between 6-OHDA + mDAP at 0 m and 4 m; the difference between 6-OHDA + mDAP at 0 m and 6 m) represents p < 0.001, and # (the difference between 6-OHDA + mDAP and 6-OHDA + vehicle at 6 m) represents p < 0.05.

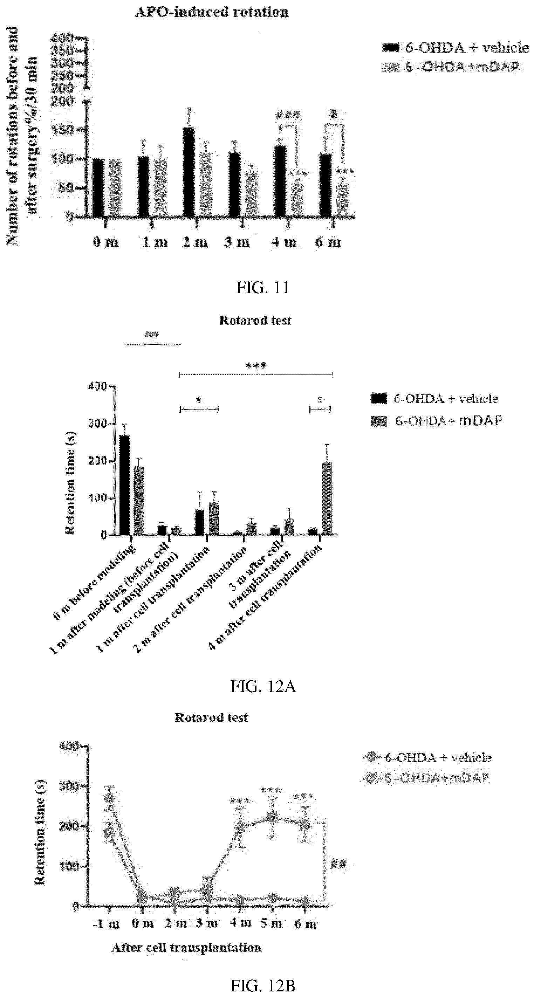

FIG. 11 is a bar chart illustrating APO rotation results before cell transplantation (1 m after modeling) and 1 m, 2 m, 3 m, 4 m, and 6 m after cell transplantation, where *** (the difference between 6-OHDA + mDAP and 6-OHDA + vehicle at 4 m and the difference between 6-OHDA + mDAP and 6-OHDA + vehicle at 6 m), and ### represent p < 0.001, and $ represents p < 0.05.
FIG. 12A is a graph illustrating rotarod test results 0 m before modeling, before cell transplantation (1 m after modeling), and 1 m, 2 m, 3 m, and 4 m after cell transplantation, where *** (the difference between 6-OHDA + mDAP before cell transplantation (1 m after modeling) and 4 m after cell transplantation), and ### (the difference between 6-OHDA + mDAP before cell transplantation (1 m after modeling) and 0 m before modeling) represent p < 0.001, * (the difference between 6-OHDA + mDAP before cell transplantation (1 m after modeling) and 1 m after cell transplantation) represents p < 0.05, and $ represents p < 0.05.
FIG. 12B is a graph illustrating rotarod test results 0 m before modeling, before cell transplantation (1 m after modeling), 2 m, 3 m, 4 m, 5 m, and 6 m after cell transplantation (corresponding to -1 m, 0 m, 2 m, 3 m, 4 m, 5 m, and 6 m in FIG. 12B, respectively), where *** represents p < 0.001 (the difference between 6-OHDA + mDAP at 0 m and 4 m; the difference between 6-OHDA + mDAP at 0 m and 5 m; the difference between 6-OHDA + mDAP at 0 m and 6 m); ## (the difference between 6-OHDA + mDAP and 6-OHDA + vehicle at 6 m) represents p < 0.01.

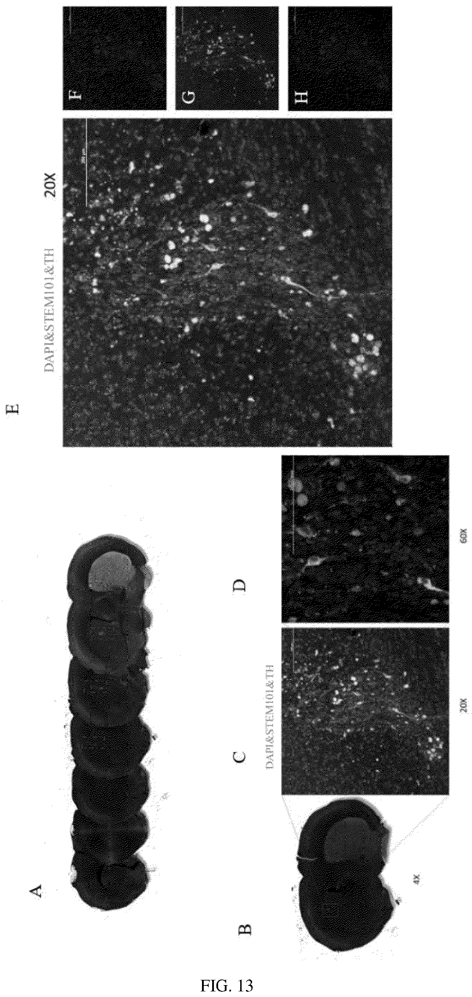

FIG. 13 is images illustrating immunofluorescence staining results of mouse brain sections 4 months after cell transplantation, where A is immunofluorescence staining images of serial brain sections 4 months after cell transplantation; B is an original image of the brain section 4 months after cell transplantation co-stained with stem101, TH, and DAPI; C is a partial enlarged view (1) of the brain section 4 months after cell transplantation co-stained with stem101, TH, and DAPI at 20 × objective lens and 10 × eyepiece magnification; D is a partial enlarged view of the brain section 4 months after cell transplantation co-stained with stem101, TH, and DAPI at 60× objective lens and 10 × eyepiece magnification; E is a partial enlarged view (2) of the brain section 4 months after cell transplantation co-stained with stem101, TH, and DAPI at 20 × objective lens and 10 × eyepiece magnification; F is a DAPI-stained region of the partial enlarged view of the brain section 4 months after cell transplantation co-stained with stem101, TH, and DAPI at 20 × objective lens and 10 × eyepiece magnification; G is a TH-stained region of the partial enlarged view of the brain section 4 months after cell transplantation co-stained with stem101, TH, and DAPI at 20 × objective lens and 10× eyepiece magnification; H is a stem101-stained region of the partial enlarged view of the brain section 4 months after cell transplantation co-stained with stem101, TH, and DAPI at 20 × objective lens and 10 × eyepiece magnification.

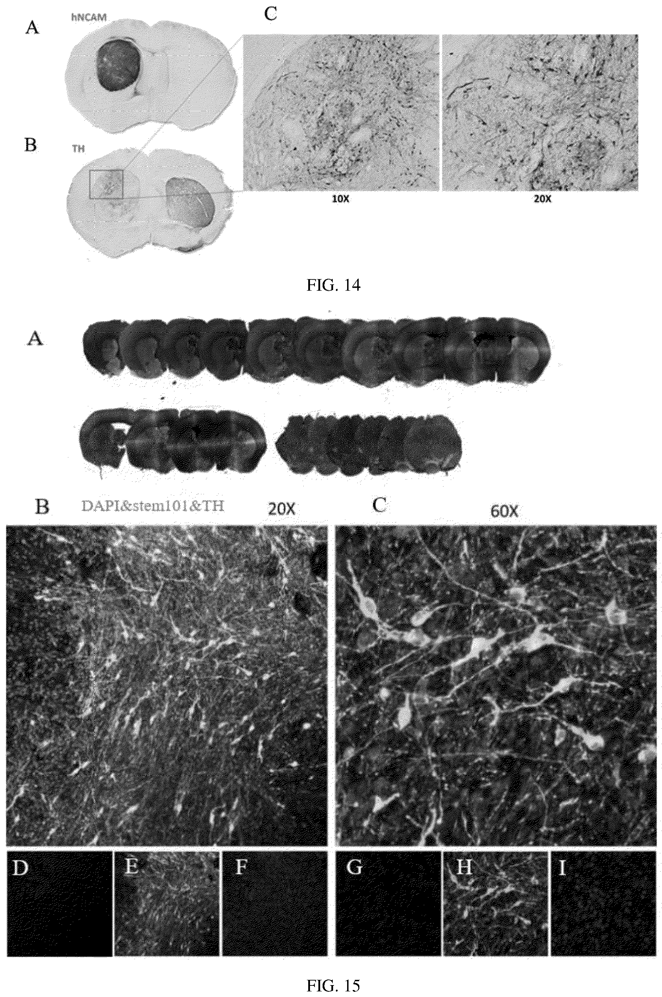

FIG. 14 is images illustrating results of IHC staining for hNCAM and TH in mouse brain sections 6 months after cell transplantation, where A is an IHC staining image for hNCAM in mouse brain sections 6 months after cell transplantation; B is an IHC staining image for TH in mouse brain sections 6 months after cell transplantation; C is a partial enlarged view of the IHC staining image for TH in mouse brain sections 6 months after cell transplantation at 10 × and 20 × magnification.
FIG. 15 is immunofluorescence staining images of mouse brain sections 6 months after cell transplantation co-stained with stem101, TH, and DAPI, where A is immunofluorescence staining images of serial mouse brain sections 6 months after cell transplantation; B is a 20 × partial enlarged view of the mouse brain section 6 months after cell transplantation co-stained with stem101, TH, and DAPI; C is a 60× partial enlarged view of the mouse brain section 6 months after cell transplantation co-stained with stem101, TH, and DAPI; D is a DAPI-stained region of the 20 × partial enlarged view of the mouse brain section 6 months after cell transplantation co-stained with stem101, TH, and DAPI; E is a TH-stained region of the 20× partial enlarged view of the mouse brain section 6 months after cell transplantation co-stained with stem101, TH, and DAPI; F is a stem101-stained region of the 20 × partial enlarged view of the mouse brain section 6 months after cell transplantation co-stained with stem101, TH, and DAPI; G is a DAPI-stained region of the 60 × partial enlarged view of the mouse brain section 6 months after cell transplantation co-stained with stem101, TH, and DAPI; H is a TH-stained region of the 60× partial enlarged view of the mouse brain section 6 months after cell transplantation co-stained with stem101, TH, and DAPI; I is a stem101-stained region of the 60× partial enlarged view of the mouse brain section 6 months after cell transplantation co-stained with stem101, TH, and DAPI; the magnifications indicated in the partial enlarged views refer to objective lens magnifications, and the eyepiece magnification in all cases is 10×.

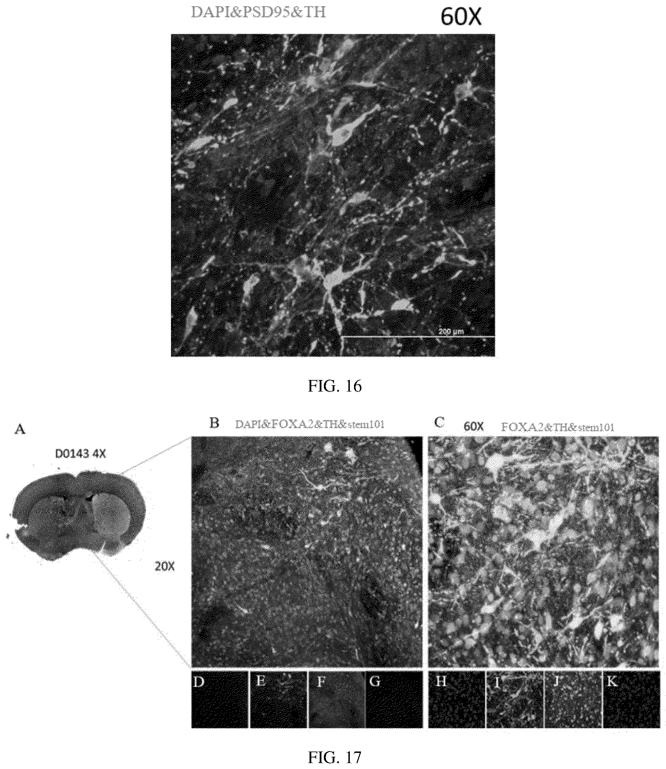

FIG. 16 is an immunofluorescence staining image of mouse brain sections 6 months after cell transplantation co-stained with PSD95, TH, and DAPI (60× refers to the objective lens magnification, and the eyepiece magnification is 10×, where the blue part is the DAPI-stained region, the yellow part is the PSD95-stained region, and the green part is the TH-stained region).
FIG. 17 is immunofluorescence staining images of mouse brain sections 6 months after cell transplantation co-stained with FOXA2, TH, stem101, and DAPI, where A is an original immunofluorescence staining image (4×) of the mouse brain section 6 months after cell transplantation co-stained with FOXA2, TH, stem101, and DAPI; B is a 20× partial enlarged view of the mouse brain section 6 months after cell transplantation co-stained with FOXA2, TH, stem101, and DAPI; C is a 60 × partial enlarged view of the mouse brain section 6 months after cell transplantation co-stained with FOXA2, TH, stem101, and DAPI; D is a DAPI-stained region of the 20 × partial enlarged view of the mouse brain section 6 months after cell transplantation co-stained with FOXA2, TH, stem101, and DAPI; E is a TH-stained region of the 20 × partial enlarged view of the mouse brain section 6 months after cell transplantation co-stained with FOXA2, TH, stem101, and DAPI; F is a FOXA2-stained region of the 20 × partial enlarged view of the mouse brain section 6 months after cell transplantation co-stained with FOXA2, TH, stem101, and DAPI; G is a stem101-stained region of the 20× partial enlarged view of the mouse brain section 6 months after cell transplantation co-stained with FOXA2, TH, stem101, and DAPI; H is a DAPI-stained region of the 60× partial enlarged view of the mouse brain section 6 months after cell transplantation co-stained with FOXA2, TH, stem101, and DAPI; I is a TH-stained region of the 60 × partial enlarged view of the mouse brain section 6 months after cell transplantation co-stained with FOXA2, TH, stem101, and DAPI; J is a FOXA2-stained region of the 60× partial enlarged view of the mouse brain section 6 months after cell transplantation co-stained with FOXA2, TH, stem101, and DAPI; K is a stem101-stained region of the 60× partial enlarged view of the mouse brain section 6 months after cell transplantation co-stained with FOXA2, TH, stem101, and DAPI; the magnifications indicated in the partial enlarged views refer to objective lens magnifications, and the eyepiece magnification in all cases is 10×.

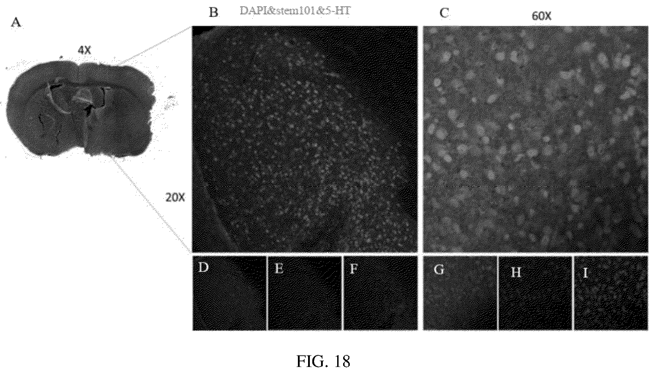

FIG. 18 is immunofluorescence staining images of mouse brain sections 6 months after cell transplantation co-stained with DAPI, stem101, and 5-HT, where A is an original immunofluorescence staining image (4×) of the mouse brain section 6 months after cell transplantation co-stained with DAPI, stem101, and 5-HT; B is a 20 × partial enlarged view of the mouse brain section 6 months after cell transplantation co-stained with DAPI, stem101, and 5-HT; C is a 60× partial enlarged view of the mouse brain section 6 months after cell transplantation co-stained with DAPI, stem101, and 5-HT; D is a 5-HT-stained region of the 20 × partial enlarged view of the mouse brain section 6 months after cell transplantation co-stained with DAPI, stem101, and 5-HT; E is a stem101-stained region of the 20× partial enlarged view of the mouse brain section 6 months after cell transplantation co-stained with DAPI, stem101, and 5-HT; F is a DAPI-stained region of the 20 × partial enlarged view of the mouse brain section 6 months after cell transplantation co-stained with DAPI, stem101, and 5-HT; G is a 5-HT-stained region of the 60 × partial enlarged view of the mouse brain section 6 months after cell transplantation co-stained with DAPI, stem101, and 5-HT; H is a stem101-stained region of the 60× partial enlarged view of the mouse brain section 6 months after cell transplantation co-stained with DAPI, stem101, and 5-HT; I is a DAPI-stained region of the 60× partial enlarged view of the mouse brain section 6 months after cell transplantation co-stained with DAPI, stem101, and 5-HT; the magnifications indicated in the partial enlarged views refer to objective lens magnifications, and the eyepiece magnification in all cases is 10×.

### DETAILED DESCRIPTION

The present disclosure will be described in further detail with reference to specific examples.

It should be understood that these examples are merely intended to illustrate the present disclosure rather than limit the scope of the present disclosure.

### Definitions

The term "stem cells" refers to cells capable of self-replication and possessing pluripotency or multipotency. Typically, stem cells can regenerate injured tissues. The stem cells used herein may be, but are not limited to, embryonic stem (ES) cells, induced pluripotent stem cells, or tissue stem cells (also referred to as tissue-specific stem cells or somatic stem cells).

"Embryonic stem (ES) cells" are pluripotent stem cells derived from early embryos. ES cells were first established in 1981, and have also been used in the production of knockout mice since 1989. In 1998, human ES cells were established, which are now becoming available for use in regenerative medicine.

Unlike ES cells, tissue stem cells have limited differentiation potential. Tissue stem cells reside at specific locations in tissues and exhibit undifferentiated intracellular structures. Therefore, tissue stem cells generally possess lower pluripotency. Tissue stem cells have a high nucleus-to-cytoplasm ratio and fewer intracellular organelles. Most tissue stem cells exhibit low pluripotency, a long cell cycle, and proliferative capacity exceeding the lifespan of the organism. Based on the origin of the cells, such as the integumentary system, digestive system, bone marrow system, and nervous system, tissue stem cells are categorized into several types. Tissue stem cells in the integumentary system include epidermal stem cells, hair follicle stem cells, and the like. Tissue stem cells in the digestive system include pancreatic (common) stem cells, hepatic stem cells, and the like. Tissue stem cells in the bone marrow system include hematopoietic stem cells, mesenchymal stem cells, and the like. Tissue stem cells in the nervous system include neural stem cells, retinal stem cells, and the like.

"Induced pluripotent stem cells" (often abbreviated as iPS cells or iPSCs) refer to a type of pluripotent stem cell that is artificially generated from non-pluripotent cells (typically somatic cells) or terminally differentiated cells (e.g., fibroblasts, hematopoietic cells, myocytes, neurons, and epidermal cells) by introducing certain factors called reprogramming factors.

The term "differentiation" refers to a process by which a less specialized cell forms progeny of at least one more new specialized cell type.

The term "basal culture medium" refers to a chemically defined culture medium, including but not limited to, basal cell culture media such as Iscove's Modified Dulbecco's Medium (IMDM), Eagle's Basal Medium (BME), MEM, DMEM, Ham's F-12 Medium, RPMI1640 Medium, Fischer's Medium, E8 Medium, mTESR Medium, StemFit Basic 03, StemFit Basic 04, NutriStem hPSC XF Medium, StemMACS iPS Brew Medium, Stem-Partner ACF Medium, TeSR-AOF Medium, and TeSR2 Medium.

The term "SB431542" also encompasses SB431542 and salts thereof, especially pharmaceutically acceptable salts thereof.

The term "CHIR99021" encompasses CHIR99021 and salts thereof, especially pharmaceutically acceptable salts thereof.

The term "Y-27632" also encompasses Y-27632 and salts thereof, especially pharmaceutically acceptable salts thereof. A preferred pharmaceutically acceptable salt is Y-27632 2HCL.

A neural progenitor cell refers to a pluripotent stem cell that has limited self-renewal capacity and is capable of differentiating into at least two distinct cell lineages. The term "dopaminergic neural progenitor cell" refers to a neural progenitor cell that is capable of producing dopamine.

The term "dopaminergic neural precursor cell" generally refers to a cell capable of proliferating and/or differentiating into dopaminergic neurons *in vitro* or *in vivo.* Dopaminergic neural precursor cells may originate from ventral midbrain nerve cells, and may be differentiated from pluripotent stem cells. Dopaminergic neural precursor cells may also be differentiated or reprogrammed from other cell types. The dopaminergic neural precursor cells in the present disclosure can be obtained through simple culturing of dopaminergic neural progenitor cells, with minimal differences in marker expression purity between the two cell types before and after conversion.

The term "midbrain floor plate cell" is a concept in neural developmental biology pertaining to the structure of the neural tube, and refers to the structure at the most ventral structure of the midbrain segment of the neural tube. Dopamine neurons develop from the midbrain floor plate. In the context of neural differentiation, midbrain floor plate cells are equivalent to dopaminergic neural progenitor cells.

The term "FOXA2" refers to a transcription factor involved in embryonic development, establishment of tissue-specific gene expression, and regulation of gene expression in differentiated tissues. It is essential for notochord formation during embryonic development. As used herein, "Foxa2" refers to the protein with Uniprot accession number: Q9Y261.

The term "PAX6" refers to a transcription factor having an important function in the development of the eye, nose, central nervous system, and pancreas. It is essential for the differentiation of pancreatic α-cells, competes with PAX4 for binding to shared elements in the promoters of glucagon, insulin, and somatostatin, and regulates the specification of ventral neuron subtypes by establishing proper progenitor cell domains. As used herein, "PAX6" refers to the protein with Uniprot accession number: P26367.

The term "OTX2" refers to a transcription factor that may be associated with the development of the brain and sensory organs. As used herein, "OTX2" refers to the protein with Uniprot accession number: P32243.

The term "En1" refers to a gene required for the proper formation of the apical ectodermal ridge and correct dorsoventral patterning of the limbs. As used herein, "En1" refers to the protein with Uniprot accession number: Q05925.

The term "HOXA2" refers to a member of the homeobox transcription factor gene family that, in vertebrates, is located within clusters named A, B, C, and D on four separate chromosomes. The expression of these proteins is spatiotemporally regulated during embryonic development. This gene is part of the A cluster on chromosome 7 and encodes a DNA-binding transcription factor that regulates gene expression, morphogenesis, and differentiation. The encoded protein may be involved in the positioning of the hindbrain segments at the appropriate location along the anterior-posterior axis during development. As used herein, "HOXA2" refers to the protein with Uniprot accession number: O43364.

Experimental methods without specified conditions in the following examples are generally conducted under conventional conditions or conditions recommended by the manufacturer. The materials, reagents, and the like used in the examples are commercially available reagents and materials unless otherwise specified.

The human pluripotent stem cells used in the examples are human induced pluripotent stem cells (iPSCs), prepared using a CTS^{™}CytoTune^{™}-iPS 2.1 Sendai virus reprogramming kit (Cat. No.: A34546) (specific methods are described in the manufacturer's instructions), and the starting cells are human fibroblasts.

### Example 1. Kit for Inducing Differentiation of Human Pluripotent Stem Cells into Dopaminergic Neural Precursor Cells

Provided was a kit for inducing differentiation of human pluripotent stem cells into dopaminergic neural precursor cells, including: a first culture medium, a second culture medium, a third culture medium, a fourth culture medium, and a fifth culture medium.

The first culture medium was a neural basal medium containing 10 µM SB431542, 2 µM DMHI1, 0.6 µM CHIR99021, and 1 µM SAG.

The second culture medium was a neural basal medium containing 10 µM Y27632, 10 µM SB431542, 2 µM DMH1, 0.6 µM CHIR99021, and 1 µM SAG.

The third culture medium was a neural basal medium containing 1 µM DMH1, 0.2 µM SAG, and 100 ng/mL fibroblast growth factor 8b (FGF8b).

The fourth culture medium was a neural basal medium containing 0.2 µM SAG and 100 ng/mL FGF8b.

The fifth culture medium was a neural basal medium containing 10 µM Y27632, 0.2 µM SAG, and 100 ng/mL FGF8b.

The neural basal media consisted of the following components: 98% (v/v) DMEM/F12 (Gibco), 1% (v/v) non-essential amino acid (Gibco), and 1% (v/v) N2 supplement (Gibco).

### Example 2-1. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of CHIR99021 in the first culture medium and the second culture medium was 0.2 µM.

### Example 2-2. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of CHIR99021 in the first culture medium and the second culture medium was 0.4 µM.

### Example 2-3. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of CHIR99021 in the first culture medium and the second culture medium was 0.8 µM.

### Example 2-4. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of CHIR99021 in the first culture medium and the second culture medium was 1.0 µM.

### Example 3-1. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of SAG in the first culture medium and the second culture medium was 0.5 µM.

### Example 3-2. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of SAG in the first culture medium and the second culture medium was 1.25 µM.

### Example 3-3. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of SAG in the first culture medium and the second culture medium was 1.5 µM.

### Example 3-4. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of SAG in the first culture medium and the second culture medium was 1.75 µM.

### Example 4. Kit for Inducing Differentiation of Human Pluripotent Stem Cells into Dopamine Neurons

Provided was a kit for inducing differentiation of human pluripotent stem cells into dopamine neurons, including: the kit for inducing differentiation of human pluripotent stem cells into dopaminergic neural precursor cells according to Example 1, and a sixth culture medium.

The sixth culture medium is a neural differentiation medium containing 20 ng/mL brain-derived growth factor (BDNF), 20 ng/mL glial cell line-derived neurotrophic factor (GDNF), 200 µM ascorbic acid (AA), 1 µM cyclic adenosine monophosphate (cAMP), and 0.1 µM compound E.

The neural differentiation medium consisted of the following components: 97.5% (v/v) Neurobasal (Gibco), 0.5% (v/v) N2 supplement (Gibco), 1% (v/v) B27 supplement (Gibco), and 1% (v/v) non-essential amino acid (Gibco).

### Example 5-1. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 2-1.

### Example 5-2. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 2-2.

### Example 5-3. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 2-3.

### Example 5-4. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 2-4.

### Example 6-1. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 3-1.

### Example 6-2. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 3-2.

### Example 6-3. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 3-3.

### Example 6-4. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Example 3-4.

### Example 7. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, including a step of using the kit of Example 4. The specific steps were as follows (a schematic diagram of the differentiation is shown in FIG. 1):
(1) Induction of differentiation of human pluripotent stem cells into dopaminergic neural precursor cells:
   1) Culture of human pluripotent stem cells:
      Human induced pluripotent stem cells were plated in the form of cell aggregates on a substrate pre-coated with laminin at about 100 thousand cells per well and cultured using an E8 complete medium. During the culture process, the cells were enzymatically dissociated and passaged every 4 to 5 days.
   2) Differentiation of dopaminergic neural precursor cells:
      S1: One day prior to differentiation, human pluripotent stem cells at a confluence of about 70% to 80% were dissociated into single cells using TrypLE enzyme, and the cells were seeded into a 6-well plate pre-coated with Vitronectin (Gibco) at a density of 8000 cells/cm². The seeding medium was StemFit complete medium supplemented with 10 µM Y27632 to enhance stem cell survival.
      S2: After 24 h of adherent growth, the medium was replaced with the first culture medium in the kit of Example 1 (designated as day 0 of differentiation, D0), and the cells were cultured in a carbon dioxide cell incubator at 37 °C with 5% CO₂ for 8 days (the medium was refreshed every two days).
      S3: On day 9 of differentiation, the cells were dissociated using Accutase (Innovative), then resuspended in the second culture medium in the kit of Example 1, and 15 million cells were seeded into a T75 suspension culture flask. After gentle agitation to ensure uniform distribution, the cells were cultured in a carbon dioxide cell incubator at 37 °C with 5% CO₂ for 2 days to allow the cells to spontaneously form neurospheres under suspension conditions, thus yielding midbrain floor plate cells.
      S4: On day 11 of differentiation, the medium was replaced with the third culture medium in the kit of Example 1, and the cells were cultured in a carbon dioxide cell incubator at 37 °C with 5% CO₂ for 5 days (the medium was refreshed every 2.5 days) to obtain dopaminergic neural progenitor cells.
      S5: On day 16 of differentiation, the medium was replaced with the fourth culture medium in the kit of Example 1, 5 million neurosphere cells were subjected to adherence treatment (adhering to a T75 culture flask pre-coated with Vitronectin (Gibco) for continuous culture), and the cells were cultured in a carbon dioxide cell incubator at 37 °C with 5% CO₂ for 7 days (the medium was refreshed every 2.5 days).
      S6: On day 23 of differentiation, the cells were dissociated using Accutase (Innovative), then resuspended in the fifth culture medium in the kit of Example 1, and 15 million cells were seeded into a T75 suspension culture flask. After gentle agitation to ensure uniform distribution, the cells were cultured in a carbon dioxide cell incubator at 37 °C with 5% CO₂ for one day to allow the cells to spontaneously form neurospheres under suspension conditions, thus yielding dopaminergic neural precursor cells (mDAP cells).
(2) Induction of differentiation of dopaminergic neural precursor cells into dopamine neurons:
   On day 24 of differentiation, the dopaminergic neural precursor cells obtained in step (1) were dissociated into single cells using Accutase, and seeded onto cell culture glass coverslips coated with Matrigel at a density of 2 × 10⁵ cells/cm². Meanwhile, the medium was replaced with the sixth culture medium in the kit of Example 4, and the cells were cultured in a carbon dioxide cell incubator at 37 °C with 5% CO₂ for 3 weeks (the medium was refreshed every 3.5 days) to obtain dopamine neurons.

### Example 8-1. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 5-1 was used.

### Example 8-2. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 5-2 was used.

### Example 8-3. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 5-3 was used.

### Example 8-4. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 5-4 was used.

### Example 9-1. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 6-1 was used.

### Example 9-2. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 6-2 was used.

### Example 9-3. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 6-3 was used.

### Example 9-4. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Example 6-4 was used.

### Comparative Example 1. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of CHIR99021 in the first culture medium and the second culture medium was 0 µM.

### Comparative Example 2. Kit for Inducing Differentiation of Human Pluripotent Stem Cells into Dopaminergic Neural Precursor Cells

Provided was a kit for inducing differentiation of human pluripotent stem cells into dopaminergic neural precursor cells, which was identical to the kit in Example 1, and only differed in that the concentration of SAG in the first culture medium and the second culture medium was 0 µM.

### Comparative Example 3. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Comparative Example 1.

### Comparative Example 4. Kit for Inducing Differentiation of Human Pluripotent Stem Cells into Dopamine Neurons

Provided was a kit for inducing differentiation of human pluripotent stem cells into dopamine neurons, which was identical to the kit in Example 4, and only differed in that the kit for inducing differentiation of human pluripotent stem cells into dopaminergic neural precursor cells was the kit in Comparative Example 2.

### Comparative Example 5. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Comparative Example 3 was used.

### Comparative Example 6. Method for Inducing Differentiation of Human Pluripotent Stem Cells into Dopaminergic Neural Precursor Cells and Dopamine Neurons

Provided was a method for inducing differentiation of human pluripotent stem cells into dopaminergic neural precursor cells and dopamine neurons, which was identical to that of Example 7, and only differed in that the kit of Comparative Example 4 was used.

### Effect Example 1. Assay of mDAP Cells Prepared by the Method of Example 7

### 1. Cell morphology

The cells at different stages of differentiation in Example 7 were taken from the incubator, observed under a microscope, and photographed for recording. The cell morphology after induction on D0, the cell morphology after induction on D7, the cell morphology after induction on D15, the cell morphology after induction on D20, the cell morphology after induction on D24, the cell morphology after induction on D26, and the cell morphology after induction on D40 are shown in A to G in FIG. 2. The cell morphologies at different induction days were consistent with the morphological characteristics of the expected midbrain floor plate cell stage, dopaminergic neural progenitor cell stage, dopaminergic neural precursor cell stage, and dopamine neuron stage. The cells differentiated at different stages in Examples 8-1 to 9-4 exhibited similar morphological characteristics.

### 2. Immunofluorescence staining

The dopaminergic neural precursor cells obtained through induction in Example 7 were washed twice with PBS after removing the supernatant. The cells were then immobilized with paraformaldehyde at room temperature for 15 min, washed three times with PBS, and permeabilized with PBST (0.2% Triton + PBS) for 10 min. After the cells were washed once with PBS, a blocking buffer (PBST + 3% donkey serum) was added and the cells were incubated for 30 min at room temperature. Subsequently, OTX2 primary antibody (1:500; R&D), FOXA2 primary antibody (1:500, R&D AF2400), EN1 primary antibody (Thermo Fisher, PA5-14149), and LMX1A primary antibody (Millipore, AB10533) were added, and the cells were incubated overnight at 4 °C and then washed 3 times with PBS. A secondary antibody (1:500; Jackson Lab) was added, and the cells were incubated at 37 °C in the dark for 1 h, washed 3 times with PBS, stained with DAPI for 5 min, washed three times with PBS, observed under a fluorescence microscope, and photographed in the dark.

The immunofluorescence staining results of DAPI, OTX2, EN1, and their combined result, as well as the immunofluorescence staining results of DAPI, FOXA2, LMX1A, and their combined result are shown in FIG. 3. It can be seen that the cell markers such as OTX2, EN1, FOXA2, and LMX1A were all highly expressed on the surface of the prepared mDAP cells, and about 90.1% of the cells co-expressed OTX2 and EN1, and about 87.5% of the cells co-expressed FOXA2 and LMX1A. The dopaminergic neural precursor cells obtained in Examples 8-1 to 9-4 exhibited similar results.

### 3. FACS assay

The mDAP cells obtained through induction in Example 7 were transferred to a 1.5 mL centrifuge tube and centrifuged to remove the supernatant. The cells were resuspended in a staining buffer (PBS containing 0.5% BSA) and evenly divided into 7 new centrifuge tubes. Isotype control antibody, OTX2 antibody, FOXA2 antibody, LMX1A antibody, CD166 antibody (Miltenyi Biotech, Cat. No.: 130-115-547), Corin antibody, and a mixture of CD166 and Corin antibodies were added to each tube, respectively. After thorough mixing, the mixture was incubated for 20 to 30 min. The cells were washed once with a staining buffer, and centrifuged to remove the supernatant. The samples were then analyzed using flow cytometry.

The results of the expression ratios of cell populations represented by the cell markers such as OTX2, FOXA2, LMX1A, CD166, and/or Corin in the dopaminergic neural precursor cells obtained in Example 7 in the total cell population are shown in FIG. 4: The proportion of the cell population specifically expressing the OTX2 marker was 97.47% in the total cell population, the proportion of the cell population specifically expressing the FOXA2 marker was 86.96% in the total cell population, the proportion of the cell population specifically expressing the LMX1A marker was 99.66% in the total cell population, the proportion of the cell population specifically expressing the CD166 marker was 97.07% in the total cell population, the proportion of the cell population specifically expressing the Corin marker was 87.74% in the total cell population, and the proportion of the cell population specifically co-expressing the CD166 and the Corin markers was 82.42% in the total cell population. The experimental data demonstrated that the proportion of CD166-positive cells on the surface of the mDAP cells prepared by the method described in Example 7 was significantly higher than the proportion of 39.12% of CD166-positive cells in the midbrain dopaminergic neural progenitor cells prepared in the Patent Application No. US20220333071A1. The dopaminergic neural progenitor cells defined in that patent application are equivalent to the dopaminergic neural precursor cells in the present disclosure, and such cells exhibit higher cell purity and lower proportions of impurity cells, which are more conducive to cell transplantation and treatment of related diseases. Similarly, the proportion of the cell population specifically co-expressing the CD166 and the Corin markers in the mDAP cells prepared by the method described in Example 7 in the total cell population was higher than that of the midbrain dopaminergic neural progenitor cells prepared in the Patent Application No. US20220333071A1, further indicating higher purity and better suitability for use in neural precursor cell transplantation and treatment of related diseases. The dopaminergic neural precursor cells obtained in Examples 8-1 to 9-4 exhibited similar results.

### Effect Example 2: Assay of Midbrain Floor Plate Cells Prepared by the Methods of Example 7, Examples 8-1 to 8-4, and Comparative Example 5

### 1. Fluorogenic quantitative PCR

The midbrain floor plate cells obtained in Example 7, Examples 8-1 to 8-4, and Comparative Example 5 were collected, and subjected to RT-QPCR on D11 to evaluate region-specific marker genes. The results are shown in FIG. 5. In FIG. 5, the Y-axis represents the relative value of the expression level of the target gene of each group relative to the internal reference gene GAPDH (the value of the gene expression level of the internal reference gene GAPDH is "1"), and the X-axis represents different doses of CHIR99021 (corresponding to Comparative Example 5, Example 8-1 to Example 8-2, Example 7, and Example 8-3 to Example 8-4, respectively).

The specific procedures were as follows: Uninduced iPSC cells and midbrain floor plate cells obtained through induction were each collected at a quantity of 5 ×10⁵ cells, and the RNA was extracted using FastPure^{®} Cell/Tissue Total RNA Isolation Kit V2 in accordance with the manufacturer's instructions. The extracted RNA was used for downstream experiments after concentration determination. 1 µg of RNA was reverse-transcribed using the HiScriptIIIRT SuperMix for qPCR(+gDNA wiper) reverse transcription kit. After reverse transcription was completed, 180 µL of DNase/RNase Free ultrapure water was added to 20 µL of system cDNA to prepare a qPCR template. A 96-well plate was used for qPCR, and the components of the fluorogenic quantitative PCR reaction system were added to the reaction system. After the addition of the reaction system in the 96-well plate was completed, the plate was sealed with a sealing film and centrifuged at 2000 rpm for 2 min. The qPCR instrument was turned on, and the parameters were set as follows: pre-incubation at 95 °C for 30 s, and
amplification at 95 °C for 10 s and 60 °C for 30 s with 40 cycles. The qPCR reaction was initiated after parameter configuration. Upon completion of the qPCR reaction, data were read and analyzed using the 2-△△CT method to calculate relative gene expression levels. The expression of OTX2, En1, and HOXA2 factors in the midbrain floor plate cells was compared using GAPDH as the internal reference gene (OTX2 was a forebrain/midbrain marker (when the expression level was high, it indicated that the cells were at the forebrain developmental stage; when the expression level decreased, it indicated that the cells were gradually transitioning from the forebrain to the midbrain lineage during development), EN1 was a midbrain marker, and HOXA2 was a hindbrain marker). The primer sequences for each factor are shown in Table 1.

**Table 1. PCR primer sequences for each factor**

| Primer | Sequence |
|---|---|
| OTX2-F1 | AGA GGA GGT GGC ACT GAA AA (SEQ ID NO:1) |
| OTX2-R1 | GCT GTT GTT GCT GTT GTT GG (SEQ ID NO:2) |
| GAPDH-F2 | ATGTTCGTCATGGGTGTGAA (SEQ ID NO:3) |
| GAPDH-R2 | AGGGGTGCTAAGCAGTTGGT (SEQ ID NO:4) |
| FOXA2-F | CCGTTCTCCATCAACAACCT (SEQ ID NO:5) |
| FOXA2-R | GGGGTAGTGCATCACCTGTT (SEQ ID NO:6) |
| HOXA2-F | CGTCGCTCGCTGAGTGCCTG (SEQ ID NO:7) |
| HOXA2-R | TGTCGAGTGTGAAAGCGTCGAGG (SEQ ID NO:8) |
| EN1-F1 | CGTGGCTTACTCCCCATTTA (SEQ ID NO:9) |
| EN1-R1 | TCTCGCTGTCTCTCCCTCTC (SEQ ID NO:10) |
| PAX6-F1 | TGGTATTCTCTCCCCCTCCT (SEQ ID NO:11) |
| PAX6-R1 | TAAGGATGTTGAACGGGCAG (SEQ ID NO:12) |

FIG. 5 shows the RNA relative expression levels of the forebrain, midbrain, and hindbrain markers from a single batch in 3 independent repeated experiments. The results of the three repeated experiments showed that when the concentration of CHIR was 0 µM, the expression level of OTX2 peaked, and when the concentration reached 1 µM, the relative expression level of HOXA2 peaked. With the increase of the concentration of CHIR99021 (0 to 1 µM), the expression of OTX2 showed a decreasing trend, the expression of HOXA2 showed an increasing trend, and the expression of EN1 showed an increase first and then a decrease, indicating that the addition of CHIR99021 promoted a posterior shift in cell fate. When the concentration of CHIR99021 reached 0.6 µM, the relative expression level of EN1 peaked. The results showed that when the concentration of CHIR99021 reached 0.6 µM, the relative expression level of the midbrain marker EN1 peaked, while the forebrain marker OTX2 and the hindbrain marker HOXA2 were both at a lower level of expression. Therefore, the optimal concentration was 0.6 µM.

### Effect Example 3: Assay of Midbrain Floor Plate Cells Prepared by the Methods of Example 7, Examples 9-1 to 9-4, and Comparative Example 6

### 1. Immunofluorescence staining

The method was the same as that in Effect Example 1.

Results and analysis: FIG. 6 shows the immunofluorescence staining results of the midbrain floor plate cells obtained through differentiation under the induction of different SAG concentrations. In the absence of SAG induction, the expression level of PAX6 marker was extremely high, while after the SAG induction, the expression level of PAX6 decreased rapidly; meanwhile, the expression level of FOXA2 gradually increased with rising concentrations of SAG, and the expression level of FOXA2 peaked when the concentration of SAG used for induction reached 1 µM (where the red fluorescence represented the immunofluorescence staining results of the marker FOXA2 of the midbrain floor plate cells obtained through differentiation, the blue fluorescence represented the immunofluorescence staining results of DAPI, and the green fluorescence represented the immunofluorescence staining results of the cell marker PAX6); when the concentration of SAG reached 1 µM to 1.75 µM, the proportion of FOXA2-positive cells reached 83% or more. The induction effect was achieved when the concentration of SAG used for induction was 1 µM to 1.75 µM, and the optimal value was 1 µM (the proportion of FOXA2-positive cells was about 85%).

### 2. Fluorogenic quantitative PCR

The method was the same as that in Effect Example 2.

### Results and analysis:

Under the induction of different SAG concentrations, the relative expression level of the marker FOXA2 of the midbrain floor plate cells obtained through differentiation is shown in FIG. 7.

The optimal concentration of SAG used for induction was 1 µM (considering both cost and induction efficiency), while good induction effect could also be achieved in the concentration range of 1 µM to 1.75 µM, and the cells obtained through differentiation exhibited high expression of FOXA2 and low expression of PAX6.

### Effect Example 4. Use of Dopaminergic Neural Precursor Cells (mDAP Cells) Prepared in Example 7 in Treating Parkinson's Disease

### 1. Experimental content

### 1.1 Basic information

Sample name: Dopaminergic neural precursor cells (mDAP cells) prepared in Example 7.

Administration route: Culturing in artificial cerebrospinal fluid (ACSF, Maokang Biotech, MX0951) for stereotactic injection into the brain.

Administration dose: 1 × 10^5 cells/µL, 2 µL per injection.

Duration of administration: Single administration.

### 1.2 Experimental animals

The information of the experimental animals is shown in Table 2.

**Table 2. Experimental animals**

| Animal species | SCID Beige mice |
|---|---|
| Source of animal | Purchased from Beijing Vital River Laboratory Animal Technology Co., Ltd. |
| Feeding status | 5 mice/cage |
| Age of animal (w) | Age at modeling: 8 w |
| | Age at cell transplantation: 12 w |

### 1.3 Modeling information

The mouse modeling information is shown in Table 3.

**Table 3. Mouse modeling information**

| **Mouse strain** | **Age (w)** | **Group** | **Sex** | **Number of mice** |
|---|---|---|---|---|
| Scid beige | 6 w (age at delivery; one-week acclimation and one-week behavioral assessment prior to modeling; official modeling at 8 w) | Vehicle (1%VC) (control group) | 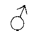 | 8 |
| | | | 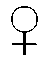 | 8 |
| | | 6-OHDA (model group) | 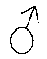 | 40 |
| | | | 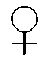 | 40 |

| | | | | |
|---|---|---|---|---|
| Note: 1. 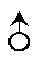 : male mouse, ♀: female mouse; 2. 6-OHDA: 6-hydroxydopamine; 3. vehicle (1% VC): vehicle group (1% ascorbic acid). | | | | |

### 1.4 Grouping information of cell transplantation

The grouping information of cell transplantation is shown in Table 4.

**Table 4. Grouping information of cell transplantation**

| **Mouse strain** | **Group** | **Sex** | **Injection sample** | **Number of mice** |
|---|---|---|---|---|
| Scid beige | 6-OHDA+ACSF (control group) | 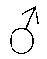 | Artificial cerebrospinal fluid | 3 |
| | | 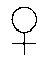 | Artificial cerebrospinal fluid | 3 |
| | 6-OHDA+mDAP (treatment group) | 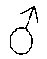 | mDAP | 5 |
| | | 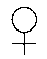 | mDAP | 5 |

| | | | | |
|---|---|---|---|---|
| Note: 1. 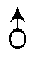 : male mouse; ♀: female mouse; 2. 6-OHDA: 6-hydroxydopamine; 3. mDAP: dopaminergic neural precursor cell. | | | | |

### 1.5 Evaluation of results

The experimental results were evaluated using a behavioral testing method.

### 1.6 Experimental procedures

### 1.6.1 Mouse modeling and screening

1) The mice were anesthetized with an isoflurane gas anesthesia machine.
2) The anesthetized mice were fixed on a mouse brain stereotaxic apparatus, and the skull surface was kept horizontal. Hair on the top of the head was removed, and the scalp was disinfected with iodophor. An incision was made in the middle of the top of the head to bluntly separate the scalp and fascia to expose the periosteum.
3) 1 µL of 6-OHDA (model group, 3 mg/mL, solvent: 1% VC) and 1 µL of 1% VC (control group) were slowly injected into the substantia nigra of the left brain of the mice by a stereotactic injection method.
4) After injection, the incision was cleaned, and sutured after ensuring no bleeding, and the mice were subjected to conventional feeding after recovery.
5) Behavioral tests were performed on week 4 after the surgery to screen model mice that met the criteria.

The behavioral tests included APO rotation and rotarod test. The specific procedures were as follows:
APO-induced rotation: The mice were intraperitoneally injected with apomorphine (APO) at 0.5 mg/kg, and after 5 min, placed into a cylinder with a diameter of 20 cm. The movement trajectory of the mice within 30 min was recorded by video, and the number of rotations within 30 min was recorded.

Rotarod test: The mice were placed on a rotarod device. On Day 1 of training, the mice were allowed to remain on the stationary rod for 10 s before the rod began accelerating from 1 rpm to 10 rpm over a period of 300 s. This process was repeated three times. On Day 2 of training, the mice were allowed to remain on the stationary rod for 15 s before the rod began accelerating from 1 rpm to 15 rpm over a period of 300 s. This process was repeated three times. On Day 3 of formal test, the mice were allowed to remain on the stationary rod for 15 s before the rod began accelerating from 1 rpm to 15 rpm. The latency to fall of the mice was recorded.

### 1.6.2 Cell transplantation

1) According to Table 4, the model mice were grouped and injected, and the injection site was the left striatum of the brain. After the surgery (cell transplantation), behavioral tests (the method was the same as that in 1.6.1) were performed once a month for 6 months.
2) Samples (mainly blood and brain tissue) were collected from the mice 4 months and 6 months after cell transplantation, and the brain tissues were subjected to immunofluorescence assay: ① The brain tissues obtained by perfusion of PFA were immersed in 20% sucrose until the tissues settled to the bottom (2 to 3 days). ② The brain tissues were immersed in 30% sucrose until the tissues settled to the bottom (2 to 3 days). ③ The dehydrated brain tissues were taken out, fixed to a sample holder with OCT compound, and rapidly frozen at a low temperature of -80 °C for storage. ④ The desired sections of 40 µm thickness were obtained. ⑤ The sections were washed in a 6-well plate containing 1 × PBS buffer, then transferred to a 96-well plate containing 150 µL of cryoprotectant (1 L of cryoprotectant: 300 g of sucrose + 400 mL of 1 × PBS buffer, volume adjusted to 1 L with ethylene glycol), and stored at -20 °C. The brain sections were retrieved from a freezer at -20 °C, and washed 3 times with 1× PBS buffer for 5 min each time. 5% donkey serum (Cat. No.: SL050, Solarbio) was added for blocking at room temperature for 1 h. Primary antibodies (mouse anti-Stem101 primary antibody, Cat. No.: Y40400, Takara; rabbit anti-TH primary antibody, Cat. No.: AB152, Millipore; goat anti-PSD95 primary antibody, Cat. No.: ab12093, Abcam; rabbit antiserum primary antibody, Sigma, Cat. No.: s5545-100 µL; goat anti-FOXA2 primary antibody, Cat. No.: AF2400, R&D) were added, and the sections were placed into a 96-well plate and incubated overnight at 4 °C. The brain sections incubated overnight at 4 °C were taken out, and the primary antibodies were recovered (stored at 4 °C). The sections were washed 3 times with 1 × PBS buffer for 5 min each time. Secondary antibodies (R(488) donkey anti-rabbit secondary antibody, Cat. No.: A-21206, Invitrogen, corresponding; G(546) donkey anti-goat secondary antibody, Cat. No.: A-11056, Invitrogen; M(647) donkey anti-mouse secondary antibody, Cat. No.: A-31571, Invitrogen) were added, and the sections were incubated at room temperature for 2 h. The sections were washed 3 times with 1 × PBS buffer for 5 min each time. DAPI (DAPI, Cat. No.: F6057, Sigma) was added dropwise, and the sections were mounted and photographed.

### 2. Experimental results

### 2.1 Behavioral test results 4 weeks after modeling

### 2.1.1 Experimental results of apomorphine (APO)-induced rotation in male and female SCID beige mice

The APO rotation results at 4 weeks after surgery (1 m after surgery) are shown in FIG. 8. The number of rotations of the mice in the model group (6-OHDA) was significantly greater than that of the control group (vehicle (1% VC), vehicle), indicating that the modeling was successful.

### 2.1.2 Experimental results of rotarod tests for male and female SCID beige mice

The rotarod test results at week 4 (1 m after surgery) are shown in FIG. 9. The duration that the model group (6-OHDA) mice remained on the rotarod was significantly shorter than that of the control group (vehicle (1% VC), vehicle) mice, indicating that the modeling was successful.

After the two behavioral tests, 40 model mice were selected, 16 of which were randomly selected for grouping and cell transplantation in the next step.

### 2.2 Behavioral test results 6 months after cell transplantation

### 2.2.1 Experimental results of apomorphine (APO)-induced rotation in male and female SCID beige mice

The APO rotation results before cell transplantation (1 m after modeling; corresponding to 0 m in FIGs. 10 and 11) and 1 m, 2 m, 3 m, 4 m and 6 m after cell transplantation (corresponding to 1 m, 2 m, 3 m, 4 m and 6 m in FIGs. 10 and 11) are shown in FIGs. 10 and 11. Six months after cell transplantation, the number of rotations of the 6-OHDA + mDAP (treatment group) mice was significantly lower than that of the 6-OHDA + ACSF (vehicle) (control group) mice; meanwhile, the number of rotations of the 6-OHDA + mDAP (treatment group) mice was significantly reduced 6 months after cell transplantation as compared to that before cell transplantation, indicating that the transplantation of the dopaminergic neural precursor cells prepared in Example 7 significantly (with statistically significant difference, p < 0.001) ameliorated the apomorphine-induced rotational behavior of the model mice.

### 2.2.2 Experimental results of rotarod tests for male and female SCID beige mice

The results of the rotarod tests 0 m before modeling, before cell transplantation (1 m after modeling), and 1 m, 2 m, 3 m, and 4 m after cell transplantation are shown in FIG. 12A. Four months after cell transplantation, the duration that the 6-OHDA + mDAP (treatment group) mice remained on the rotarod was significantly longer than that of the 6-OHDA + ACSF (control group) mice; meanwhile, the duration that the 6-OHDA + mDAP (treatment group) mice remained on the rotarod was significantly increased 4 months after cell transplantation as compared to that before cell transplantation. The results of the rotarod tests 0 m before modeling, before cell transplantation (1 m after modeling), and 2 m, 3 m, 4 m, 5 m, and 6 m after cell transplantation (corresponding to -1 m, 0 m, 2 m, 3 m, 4 m, 5 m, and 6 m in FIG. 12B, respectively) are shown in FIG. 12B, indicating that the transplantation of the dopaminergic neural precursor cells prepared in Example 7 significantly ameliorated postural and gait dysfunction in the model mice.

### 3.2.3 Immunofluorescence staining results of mouse brain sections 4 months after cell transplantation

Coronal frozen sections of brain tissues from the mice 4 months after cell transplantation were co-stained with stem101 (red, H in FIG. 13), TH (green, G in FIG. 13), and DAPI (blue, F in FIG. 13) (B in FIG. 13 is an original image of the brain section, C in FIG. 13 and E in FIG. 13 are partial enlarged views of B in FIG. 13 at 20 × objective lens and 10 × eyepiece magnification, and D in FIG. 13 is a partial enlarged view of B in FIG. 13 at 60× objective lens and 10× eyepiece magnification). The survival and directed differentiation of the transplanted cells were determined 4 months after cell transplantation. A relatively large number of the transplanted cells were observed to survive, and some of them underwent directed differentiation into dopaminergic neurons. (A in FIG. 13 shows, from left to right, immunofluorescence staining images of serial brain sections post-cell transplantation.)

### 3.2.4 Immunofluorescence staining and immunohistochemical results of mouse brain sections 6 months after cell transplantation

Coronal frozen sections of brain tissues from the mice 6 months after cell transplantation were subjected to immunohistochemical (IHC) staining for hNCAM (primary antibody incubation: the brain sections were taken from a freezer at -20 °C, and tissue areas were circled using an immunohistochemical pen; 3% H₂O₂ was added, and the system was incubated for 15 min in the dark; the sections were washed 3 times with 1 × PBS buffer for 5 min each time; 5% donkey serum was added for blocking at room temperature for 1 h; a hNCAM primary antibody (Sc-106, Abcam) or a TH primary antibody (Millipore, AB152) was added, followed by incubation in a humidified chamber at 4 °C overnight. The brain sections incubated overnight at 4 °C were taken out, and the primary antibody was recovered (stored at 4 °C), followed by washing 3 times with 1 × PBS buffer for 5 min each time. The secondary antibody 5% donkey anti-mouse serum was added, followed by incubation at room temperature for 1 h. The brain sections were washed 3 times with 1 × PBS buffer for 5 min each time, and DAB color development solution was added for color development. When obvious color development reaction could be seen, the brain sections were washed 3 times with 1 × PBS buffer for 5 min each time to stop the reaction. The brain sections were air-dried at room temperature overnight, and the completely air-dried brain sections appeared white.), and the number of transplanted human-derived cells 6 months after injection of the mDAP cells was determined. The results, shown in FIG. 14, demonstrated that a relatively large number of the transplanted cells survived in the red box, and had undergone extensive differentiation into dopaminergic neurons. Coronal frozen sections of brain tissues from the mice 6 months after cell transplantation were subjected to immunofluorescence co-staining with stem101 (red), TH (green), and DAPI (blue) to determine the amount of residual transplanted cells and the quantity of differentiated dopaminergic neurons 6 months after cell transplantation. The results, shown in FIG. 15, demonstrated that a relatively large number of the transplanted cells survived, and some of them underwent directed differentiation into dopaminergic neurons. Coronal frozen sections of brain tissues from the mice 6 months after cell transplantation were co-stained with PSD95 (yellow), TH (green), and DAPI (blue), and the results, shown in FIG. 16, demonstrated that synaptic connections were generated between differentiated dopaminergic neurons. Coronal frozen sections of brain tissues from the mice 6 months after cell transplantation were co-stained with FOXA2 (yellow), TH (green), stem101 (red), and DAPI (blue), and the results, shown in FIG. 17, demonstrated that most of the mDAP cells injected into the brain were co-labeled with FOXA2, indicating a potential for differentiation into dopaminergic neurons. Coronal frozen sections of brain tissues from the mice 6 months after cell transplantation were co-stained with DAPI (blue), stem101 (red), and 5-HT (green) to quantify the differentiation of DAP cells into non-specifically differentiated 5-HT neurons. The results, shown in FIG. 18, demonstrated no co-localization between the non-specifically differentiated 5-HT neurons and the transplanted mDAP cells, indicating that the mDAP cells exhibited extremely high purity and strong differentiation specificity, and did not differentiate into off-target 5-HT neurons.

The examples described above are preferred embodiments of the present disclosure, which, however, are not intended to limit the embodiments of the present disclosure. Any other changes, modifications, substitutions, combinations, and simplifications can be made without departing from the spirit and principle of the present disclosure, and should be construed as equivalent replacements and included in the protection scope of the present disclosure.

## Claims

1. A kit, comprising a first culture medium, a second culture medium, a third culture medium, a fourth culture medium, and a fifth culture medium, wherein the first culture medium is a basal culture medium comprising a GSK3β inhibitor, an SHH agonist, and at least one or at least two of the following: a TGFβ/ALK inhibitor and a BMP4 inhibitor;
the second culture medium is a basal culture medium comprising a GSK3β inhibitor, an SHH agonist, and at least one, at least two, or at least three of the following: a ROCK inhibitor, a TGFβ/ALK inhibitor, and a BMP4 inhibitor;
the third culture medium is a basal culture medium comprising at least one, at least two, or at least three of the following: a BMP4 inhibitor, an SHH agonist, and a growth factor;
the fourth culture medium is a basal culture medium comprising at least one or at least two of the following: an SHH agonist and a growth factor;
the fifth culture medium is a basal culture medium comprising at least one, at least two, or at least three of the following: a ROCK inhibitor, an SHH agonist, and a growth factor.

2. The kit according to claim 1, wherein
the TGFβ/ALK inhibitors of the first culture medium and the second culture medium are each independently selected from at least one of: SB431542, SB-505, A-83-01, GW6604, IN-1130, Ki26894, LY2157299, LY364947, LY550410, LY573636, LY580276, NPC-30345, SB-505124, SD-093, Sm16, SM305, SX-007, Antp-Sm2A, and LY2109761;
preferably, the BMP4 inhibitors of the first culture medium, the second culture medium, and the third culture medium are each independently selected from: at least one of dorsomorphin, noggin, LDN-193189, follistatin, chordin, gremlin, and DMH1; preferably, the GSK3β inhibitors of the first culture medium and the second culture medium are each independently selected from: at least one of GSK3β inhibitor IX, SB216763, GSK3β inhibitor VII, L803-mts, 6-bromo-indirubin-3'-oxime, TWS119, AZD2858, AR-A014418, TDZD-8, LY2090314, 2-D08, IM-12, 1-azakenpaullone, indirubin, and CHIR99021;
preferably, the SHH agonists of the first culture medium, the second culture medium, the third culture medium, the fourth culture medium, and the fifth culture medium are each independently selected from: at least one of SHH, SHH C25II, SAG, SAG 21K, Hh-Ag1.5, 20a-hydroxycholesterol, and purmorphamine;
preferably, the ROCK inhibitors of the second culture medium and the fifth culture medium are each independently selected from: at least one of Y-27632, HA100, HA1152, blebbistatin, HA-1077, KD-025, Y-33075, and narciclasine;
preferably, the growth factors of the third culture medium, the fourth culture medium, and the fifth culture medium are each independently selected from at least one of epidermal growth factor, platelet-derived growth factor, fibroblast growth factor, hepatocyte growth factor, insulin-like growth factor-I, IGF-II, leukemia inhibitory factor, nerve growth factor, oncostatin M, platelet-derived endothelial cell growth factor, transforming growth factor-α, and vascular endothelial cell growth factor;
preferably, the basal culture media of the first culture medium, the second culture medium, the third culture medium, the fourth culture medium, and the fifth culture medium are each independently selected from at least one of IMDM, Eagle's Basal Medium, GMEM, MEM, DMEM, Ham's F-12 Medium, RPMI1640 Medium, and Neurobasal Medium.

3. The kit according to claim 2, wherein
the kit further comprises a sixth culture medium, and the sixth culture medium is a basal culture medium comprising a brain-derived growth factor, a glial cell line-derived neurotrophic factor, ascorbic acid, cyclic adenosine monophosphate, and compound E;
preferably, the basal culture medium of the sixth culture medium comprises at least one of IMDM, BME, GMEM, MEM, DMEM, Ham's F-12 Medium, RPMI1640 Medium, and Neurobasal Medium.

4. Use of the kit according to claims 1 to 3 in any one of (1) to (4):
(1) preparing dopaminergic neural precursor cells; (2) preparing dopamine neurons; (3) preparing a product for inducing differentiation of stem cells into dopaminergic neural precursor cells; and (4) preparing a product for inducing differentiation of stem cells into dopamine neurons.

5. Any one of the following methods a1) and a2):
a1) a method for inducing differentiation of stem cells into dopaminergic neural precursor cells, comprising a step of using the kit according to any one of claims 1 to 3;
a2) a method for inducing differentiation of stem cells into dopamine neurons, comprising a step of using the kit according to any one of claims 1 to 3.

6. The method according to claim 5, wherein
the method for inducing differentiation of stem cells into dopaminergic neural precursor cells in a1) comprises the following steps:
(1) subjecting the stem cells to a first culture using the first culture medium in the kit according to any one of claims 1 to 3, then resuspending the stem cells in the second culture medium in the kit according to any one of claims 1 to 3, and performing a second culture to obtain midbrain floor plate cells;
(2) subjecting the midbrain floor plate cells to a third culture using the third culture medium in the kit according to any one of claims 1 to 3 to obtain dopaminergic neural progenitor cells; and
(3) subjecting the dopaminergic neural progenitor cells to a fourth culture using the fourth culture medium in the kit according to any one of claims 1 to 3, and then to a fifth culture using the fifth culture medium in the kit according to any one of claims 1 to 3 to obtain dopaminergic neural precursor cells; or
the method for inducing differentiation of stem cells into dopamine neurons in a2) comprises the following steps:
(1) subjecting the stem cells to a first culture using the first culture medium in the kit according to any one of claims 1 to 3, then resuspending the stem cells in the second culture medium in the kit according to any one of claims 1 to 3, and performing a second culture to obtain midbrain floor plate cells;
(2) subjecting the midbrain floor plate cells to a third culture using the third culture medium in the kit according to any one of claims 1 to 3 to obtain dopaminergic neural progenitor cells;
(3) subjecting the dopaminergic neural progenitor cells to a fourth culture using the fourth culture medium in the kit according to any one of claims 1 to 3, and then to a fifth culture using the fifth culture medium in the kit according to any one of claims 1 to 3 to obtain dopaminergic neural precursor cells; and
(4) subjecting the dopaminergic neural precursor cells to a sixth culture using the sixth culture medium in the kit according to claim 3 to obtain dopamine neurons;
preferably, the first culture in step (1) of a1) or a2) is performed for 6 to 10 days;
preferably, the second culture in step (1) of a1) or a2) is performed for 1 to 3 days;
preferably, the third culture in step (2) of a1) or a2) is performed for 3 to 7 days;
preferably, the fourth culture in step (3) of a1) or a2) is performed for 5 to 9 days;
preferably, the fifth culture in step (3) of a1) or a2) is performed for 0.5 to 1.5 days;
preferably, the sixth culture in step (4) of a2) is performed for 18 to 24 days.

7. A dopaminergic neural precursor cell, wherein the dopaminergic neural precursor cell is prepared using the kit according to any one of claims 1 to 3 and/or the method for inducing differentiation of stem cells into dopaminergic neural precursor cells according to any one of claims 5 to 6;
preferably, a content of OTX2+EN1+ cells in the dopaminergic neural precursor cell is greater than 80%;
preferably, a content of FOXA2+LMX1A+ cells in the dopaminergic neural precursor cell is greater than 80%;
preferably, a content of CD166+ cells in the dopaminergic neural precursor cell is greater than 80%;
preferably, a content of Corin+ cells in the dopaminergic neural precursor cell is greater than 80%.

8. A dopamine neuron, wherein the dopamine neuron is prepared using the kit according to any one of claims 1 to 3 and/or the method for inducing differentiation of stem cells into dopamine neurons according to any one of claims 5 to 6.

9. A pharmaceutical composition, comprising the dopaminergic neural precursor cell according to claim 7 and/or the dopamine neuron according to claim 8, wherein
preferably, the pharmaceutical composition further comprises a pharmaceutically acceptable carrier or auxiliary material;
preferably, the pharmaceutical composition further comprises other drugs for preventing and/or treating a nervous system disease.

10. Any one of the following use b1) and b2):
b1) use of the dopaminergic neural precursor cell according to claim 7 and/or the dopamine neuron according to claim 8 in constructing a cell bank;
b2) use of the dopaminergic neural precursor cell according to claim 7, the dopamine neuron according to claim 8, and/or the pharmaceutical composition according to claim 9 in preparing a medicament for preventing and treating a nervous system disease.
